# EUROPEAN PATENT APPLICATION

(11) **EP 2 044 958 A2**
(43) Date of publication of application: **08.04.2009**
(21) Application number: 08021793.8
(22) Date of filing: 19.03.2002
(51) Int. Cl.: A61K 45/00, A61K 31/4985, A61K 31/403, A61K 31/437, A61K 31/404, A61P 43/00, A61P 9/10

(54) **Pharmaceutical composition for treating arteriosclerosis**

(30) Priority: 19.03.2001 JP 2001078569; 28.12.2001 JP 2001401289
(62) Divisional of application: 02705327.1
(71) Applicant: Shionogi & Co., Ltd., Osaka 541-0045 (JP)
(72) Inventor: Saiga, Akihiko, Osaka-shi Osaka 553-0002 (JP); Ono, Takashi, Osaka-shi Osaka 553-0002 (JP); Yamada, Katsutoshi, Osaka-shi Osaka 553-0002 (JP); Hanasaki, Kohji, Osaka-shi Osaka 553-0002 (JP)
(74) Representative: Hayes, Adrian Chetwynd

(57) **Abstract**

A pharmaceutical composition comprising a group V or X sPLA₂-inhibiting compound represented by general formula (I) : 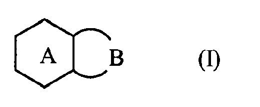 wherein
ring A is: 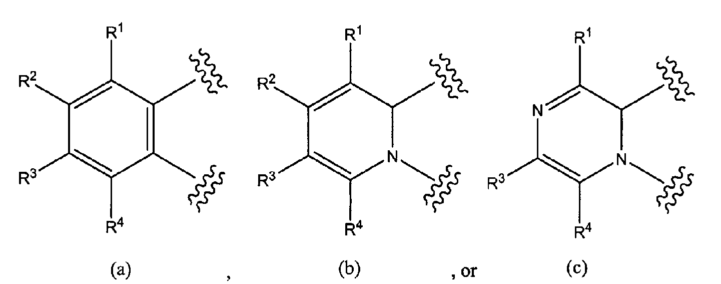 -B- is a group of (e)-(h): 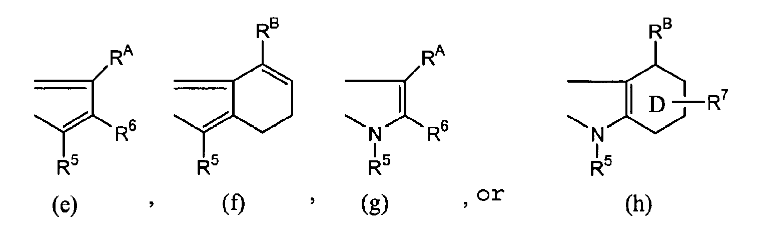 for use in suppressing degeneration of serum lipoproteins and/or treating or preventing arteriosclerosis and/or treating or preventing an ischemic disease based on arteriosclerosis.

## Description

### FIELD OF THE INVENTION

The present invention relates to group V or X secretory phospholipase A₂ inhibitors that inhibit potent degeneration activity of group V or X secretory phospholipase A₂ (sPLA₂) on serum lipoproteins, and, more particularly, to pharmaceutical compositions for treating, preventing, and diagnosing an ischemic disease based on arteriosclerosis.

### BACKGROUND ART

Arteriosclerosis is a general term that means a pathology wherein the artery wall thickens and hardens. Arteriosclerosis lesion is pathologically classified into the three kinds; atherosclerosis, arteriolar arteriosclerosis and medial necrosis, and, among them, atherosclerosis has been emphasized in the causes of ischemic diseases such as myocardial infarction and cerebral infarction. Hyperlipidemia, which is a condition wherein the level of serum lipids such as cholesterol and neutral fats in serum is increased, is closely associated with the development of atherosclerosis. Serum lipids occur as lipoproteins that form complexes with a protein. Specifically, lipoproteins containing cholesterol are classified depending on the density into very low density lipoprotein (VLDL), low density lipoprotein (LDL), high density lipoprotein (HDL), and the like. LDL among them is taken up by cells in peripheral tissues via LDL receptor, and is responsible for deliver of cholesterol into the cells.

LDLs in the initial stage of atherosclerosis are degenerated in any way to induce peroxidized lipids, increased negative charge of the whole particles, composition altered by phospholipolysis and structure alteration involved, diminished size (increase in the density), and the like, compared to normal LDLs (Yokode, et al., Rinshokensa 44, 1052-1058 (2000)), and the degenerated LDLs are taken up by monocytes and macrophage that infiltrate into vascular intimal tissues via scavenger receptors, thereby depositing oily droplets of cholesteryl esters in foam cells, and accelerating the formations of fatty streak in association with T lymphocytes and vascular smooth muscle. Thereafter, the pathology proceeds by interaction between the cells residing in the fatty streak so as to form rigid protruded lesions in vascular lumens, called fibrous plaque, and then develops into atherosclerosis, which is associated with calcification and thrombotic events, with increasing the surrounding connective tissues. On the other hand, HDLs play a role in delivering excessive cholesterol of peripheral tissues into liver, and are responsible for suppression of arteriosclerosis development. Thus, in case that HDL level in blood is depressed or that HDL is degenerated, then HDL functions are lowered, and the level of cholesterol released in blood is increased, which is one of the etiological factors in arteriosclerosis.

As shown above, degeneration or qualitative alteration of lipoproteins such as LDL and HDL is one of the major etiological factors in the development of arteriosclerosis. As one of the degeneration, oxidative modification has been known, in which the oxidized LDLs are increased in negative charge, diminished, and increased in the density. The oxidative modification has been believed responsible for the development of arteriosclerosis pathology, because the oxidized LDLs are detected at the actual lesions of arteriosclerosis, and because LDLs artificially oxidized induce foaming of macrophage, and activate the cells to promote the production of various cytokines.

On the other hand, the phospholipid composition in degenerated LDLs has been found to be altered (phospholipolysis), and thus this finding has focused attention on a phospholipolytic enzyme, phospholipase A₂ (PLA₂) as a molecule responsible for the degeneration of LDL and HDL. Phospholipase A₂ (PLA₂; EC 3.1.1.4) is a general term that represents a phospholipolytic enzyme that hydrolyzes the 2-acyl ester bond of 3-sn-phosphoglycerides. Among them, secretory PLA₂ (sPLPL₂) represents PLA₂ molecules having a lower molecular weight (13-18 kDa) that are extracellularly secreted, and are known to include eight kinds of group IB, IIA, IID, IIE, IIF, V, X, and XII in human. Any one of the molecular species contains 12 to 16 Cys residues in its structure, which form intramolecular disulfide bonds, and contains the conserved active center composing of His-Asp residues. They also contain the common Ca²⁺ -binding site, and require a Ca²⁺ level of mM order to express the enzyme activity (Ishizaki, et al., J. Biol. Chem. 274, 24973-24979 (1999); Suzuki, et al., J. Biol. Chem. 275, 5785-5793 (2000); Six, et al., Biochim. Biophys. Acta 1488, 1-19 (2000); and Gelb, et al., J. Biol. Chem. 275, 39823-39826 (2000)). Group IIA sPLA₂ was found to be expressed at vascular smooth muscles and foam cells in human arteriosclerosis lesions, and the expression has been recognized to have a correlation with the development of arteriosclerosis (Elinder, et al., Arterioscler. Thromb. Vasc. Biol. 17, 2257-2263 (1997), and Menschikowski, et al., Atherosclerosis 118, 173-181 (1995)). Further, transgenic mice that express a high level of human group IIA sPLA₂ were found to be increased in LDL level and decreased in HDL level, and to have arteriosclerosis lesions (Tietge, et al., J. Biol. Chem. 275, 10077-10084 (2000)), as well as to deteriorate the arteriosclerosis compared to normal mice when a high-fat diet is administered (Ivandic, et al., Arterioscler. Thromb. Vasc. Biol.19, 1284-1290 (1999)), thereby group IIA sPLA₂ drawing attention in the contribution to arteriosclerosis development. Also, it have been found that LDLs treated with sPLA₂ from bee venom are increased in negative charge of the whole particles, altered in phospholipid composition, and increased in the uptake into macrophage (Aviram, et al., Biochem. Biophys. Res. Commun. 185, 465-472 (1992)), and that they have characterizations very similar to small dense LDLs that are considered as one of risk factors of ischemic diseases (Sartipy, et al., J. Biol. Chem. 274, 25913-25920 (1999)). Further, it has been known that HDLs treated with sPLA₂ from snake venom are altered in the size and density in association with phospholipolysis, and that they are increased in the uptake by hepatocytes (Collet, et al., Biochim. Biophys. Acta 1043, 301-310 (1990)). Although, among the human sPLA₂ molecules, group IIA sPLA₂ particularly has a hydrolyzing potency for phospholipids of lipoproteins as described above, group IIA sPLA₂ has been found to be extremely weaker in phospholipolysis for LDL than the sPLA₂ from bee venom (Hurt-Camejo, et al., Curr. Opin. Lipidol. 11, 465-471 (2000)), and to be stronger in phospholipolysis for HDL than that for LDL (Pruzanski, et al., J. Lipid. Res. 39, 2150-2160 (1998)). In view of these findings, sPLA₂ molecular species other than group IIA sPLA₂ would be responsible for arteriosclerosis development, and it is unknown which endogenous sPLA₂ molecule would be responsible for arteriosclerosis development, showing that the exact degeneration activity of these sPLA₂ molecules on lipoproteins and the exact expression profiles thereof in arteriosclerosis have been unknown.

Human group X sPLA₂ was cloned from fetal lung tissues in 1997 on the basis of sPLA₂ -related sequences of the DNA database (Cupillard, et al., J. Biol. Chem. 272, 15745-15752 (1997)). Gene of human group X sPLA₂ is located on chromosome 16 differently from the other sPLA₂ s. The enzyme contains 16 cysteine residues in the molecule, and contains all of the intramolecular disulfide linkages located at positions characteristic of both group IB sPLA₂ and group II (groups IIA/IID/IIE/IIF) sPLA₂. Additionally, the enzyme possesses a carboxyl terminus-based extension structure that is unique of group II sPLA₂. Group X sPLA₂ exists in two molecular species, one of which is an inactivated form, pro-form, that possesses a propeptide sequence consisting of 11 amino acid residues attached at the N-terminus, and the other of which is an activated form that is produced by cleavage and deletion of the propeptide sequence with a protease such as trypsin (Morioka, et al., Arch. Biochem. Biophys. 381, 31-42 (2000)). Activated group X sPLA₂ has been shown to have a more potent enzymatic activity than other sPLA₂ s from mammals such as group IB and IIA sPLA₂ irrespective of the kind and the property of substrates, phospholipids, and further to have much more potent activities which release arachidonic acids from the cell membranes at the contact to the cells, and which produce lipid mediators such as eicosanoids and lysophospholipids, than group IB and IIA sPLA₂ (Hanasaki, et al., J. Biol. Chem. 274, 34203-34211 (1999); Saiga, et al., Biochim. Biophys. Acta 1530, 67-76 (2001)). Immunohistochemical analysis using the specific antibodies revealed that group X sPLA₂ is expressed in type II alveolar epithelial cells and splenic macrophages, and is accelerated in the expression in human colon cancer cells, suggesting that the enzyme would be responsible for inflammatory and immunological responses, as well as the onset and development of colon cancers (Morioka, et al., FEBS Lett. 487, 262-266 (2000)). However, since no report describes the degeneration activity of group X sPLA₂ on lipoproteins and the expression thereof at the arteriosclerosis lesions, it has been unknown that the enzyme would be responsible for the onset and development of arteriosclerosis.

Human group V sPLA₂ was cloned in 1994 (Chen , et al., J. Biol.Chem., 1994, 269, 2365-2368). Gene of human group V sPLA₂ is located on chromosome 1 as same as group IIA sPLA₂. The enzyme contains 12 cysteine residues in the molecule, which is less than group IIA sFLA₂ by two residues, and contains no carboxyl terminus-based extension structure found in group II sPLA₂. The expression of group V sPLA₂ is detected mainly in heart, as well as lung and inflammatory cells such as mast cells and macrophages, and, in such cells, the enzyme is known to involve the production of lipid mediators (Makoto Murakami, Ichirou Kudou, Gendaiiryou, 2000, 32, 517-548). However, since no report describes the degeneration activity of group V sPLA₂ on lipoproteins, it has been unknown that the enzyme would be responsible for the onset and development of arteriosclerosis.

### DISCLOSURE OF THE INVENTION

The inventors of the present application investigated physiological activities of human group V or X sPLA₂, and found that these enzymes exhibit a potency that powerfully releases fatty acids from the phospholipid comprised in lipoproteins in blood.

On the basis of the finding, the inventors demonstrated that the compounds that inhibit group V and/or X sPLA₂ suppress the degeneration activity of group V or X sPLA₂ on serum lipoproteins, thus accomplishing the present invention.

For compounds that inhibit sPLA₂, EP-620214 (Japanese Patent Publication (kokai) No. 7-010838, US-5578634), EP-620215 (Japanese Patent Publication (kokai) No. 7-025850, US-5684034), EP-675110 (Japanese Patent Publication (kokai) No. 7-285933, US-5654326), WO96/03120 (Japanese Patent Publication (kokai) No. 10-505336), WO96/03376 (Japanese Patent Publication (kokai) No. 10-503208, US-5641800), WO96/03383 (Japanese Patent Publication (kokai) No. 10-505584), WO97/21664 (EP-779271), WO97/21716 (EP-779273), WO98/18464 (EP839806), WO98/24437 (EP846687), WO98/24756, WO98/24794, WO98/25609, WO99/51605, and WO99/59999 describe some compounds; Roberts M.F., et al., J. Biol. Chem. 1977, 252, 2405-2411 describes p-bromophenacyl bromide; Vadas P., et al., Lab. Invest. 1986, 55, 391-404 describes mepacrine; Lombardo D., et al., J. Biol. Chem. 1985, 260, 7234-7240 describes Manoalide; and Yoshida T., et al., J. Antibiotics. 1991, 44, 1467-1470 describes thielocin A1; and Hanasaki K., et al., J.Biol.Chem. 1999 Nov 26; 274 (48): 34203-11 and Suzuki N, , et al., J.Biol.Chem. 2000 Feb 25; 275 (8): 5785-93 describe indoxam. However, no publications describe that compounds that inhibit sPLA₂ as disclosed therein have any inhibitory activity on the degeneration of serum lipoproteins.

The present invention has been accomplished on the basis of the findings as shown above.

Specifically, the present invention relates to:
(1) A pharmaceutical composition for suppressing degeneration of serum lipoproteins, a pharmaceutical composition for treating or preventing arteriosclerosis, or a pharmaceutical composition for treating or preventing an ischemic disease based on arteriosclerosis, which comprises a group V and/or X sPLA₂-inhibiting compound as an active ingredient;
(2) The pharmaceutical composition of the present invention, in which the group V and/or X sPLA₂-inhibiting compound is shown by general formula (I): 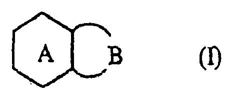 in which
   ring A is: 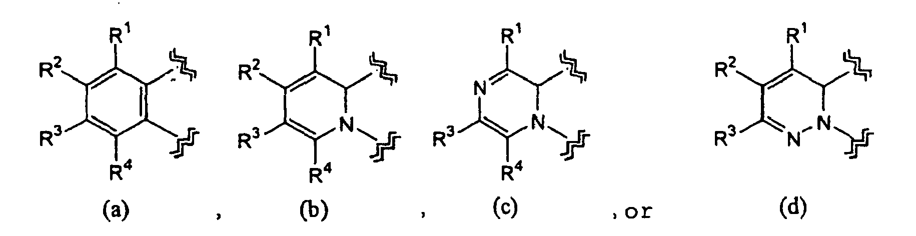 in which either one of R¹ and R² is a group of formula: -(L¹)-(acidic group) wherein L¹ is a linker group to the acidic group, and the length of the linker is 1-5, and the other is a hydrogen atom, a non-interfering substituent, or -(L')-(acidic group) wherein L¹ is as defined above; and
   each R³ and R⁴ is independently a hydrogen atom, a non-interfering substituent, a carbocyclic group, a carbocyclic group substituted by a non-interfering substituent, a heterocyclic group, or a heterocyclic group substituted by a non-interfering substituent; and
   -B- is a group of (e)-(h): 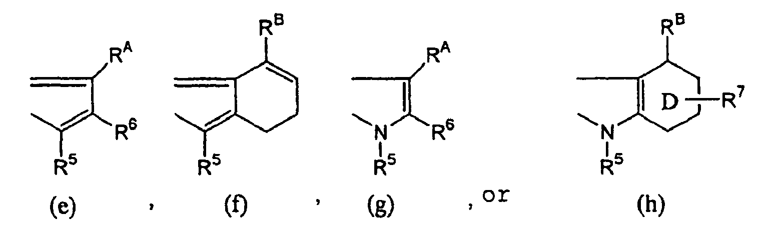 in which R⁵ is a substituent selected from a group consisting of (j) a group of a C1-C20 alkyl, a C2-C20 alkenyl, a C2-C20 alkynyl, a carbocyclic group, or a heterocyclic group; (k) a group of (j) as described above that is substituted by one or more non-interfering substituents each of which is independently selected; or a group of formula: -(L²)-R⁸
   in which L² is a divalent linker group of 1-18 atoms selected from a hydrogen atom, a nitrogen atom, a carbon atom, an oxygen atom and a sulfur atom, and R⁸ is a group selected from (j) and (k);
   R⁶ is a hydrogen atom, a halogen, a C 1-C3 alkyl, a C3-C4 cycloalkyl, a C3-C4 cycloalkenyl, a C1-C3 alkyloxy, or a C1-C3 alkylthio;
   R⁷ is a hydrogen atom or a non-interfering substituent;
   R^{A} is a group of formula: 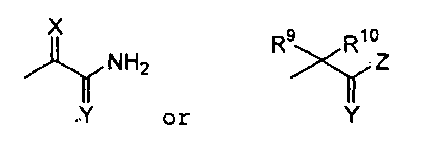 in which each R⁹ and R¹⁰ is independently a hydrogen atom, a C1-C3 alkyl, or a halogen; each X and Y is independently an oxygen atom or a sulfur atom; and Z is -NH₂ or -NHNH₂;
   R^{B} is -CONH₂ or -CONHNH₂; and
   ring D is a cyclohexene ring or a benzene ring, provided that, when -B- is a group of (e) or (f), then ring A is a ring of (b), (c), or (d); or a prodrug thereof or a pharmaceutically acceptable salt of them or a solvate of them;
   Preferably, the pharmaceutical composition of the present invention, in which the group V and/or X sPLA₂-inhibiting compound is shown by general formula (I) in which
   R¹ is a hydrogen atom or a group of formula: -(L³)-R¹¹ wherein L³ is -OCH₂-, -SCH₂-, -NH-CH₂-, -CH₂-CH₂-, -O-CH(CH₃)-, or -O-CH-(CH₂CH₂C₆C₅)-; and R¹¹ is -COOH, -CONHSO₂C₆H₅, -SO₃H, or -P(O)(OH)₂; and
   R² is a hydrogen atom or a group of formula: -(L⁴)-R¹² wherein L⁴ is a group of formula: 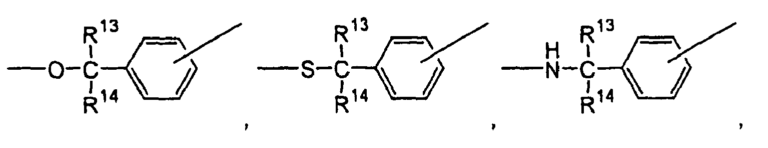 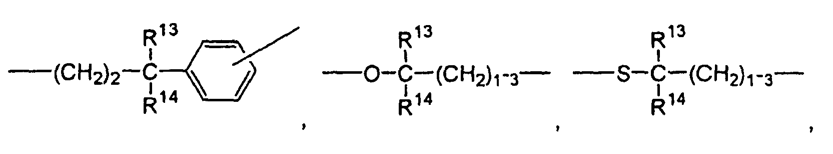 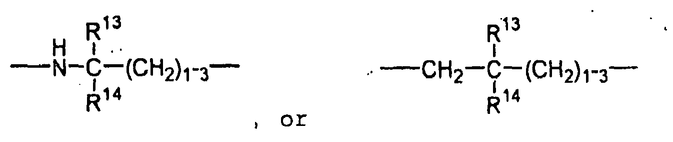 in which each R¹³ and R¹⁴ is independently a hydrogen atom, a C1-C10 alkyl, a C1-C10 aralkyl, a carboxy, an alkyloxycarbonyl, or a halogen; and R¹² is -COOH, -SO₃H, or -P(O)(OH)₂,
   provided that R¹ and R² are not a hydrogen atom, simultaneously; or a prodrug thereof or a pharmaceutically acceptable salt of them or a solvate of them;
   More preferably, the pharmaceutical composition of the present invention, in which the group V and/or X sPLA₂-inhibiting compound is shown by general formula (I) in which
   R³ is a hydrogen atom, a C1-C6 alkyl, a C3-C6 cycloalkyl, an aryl, or a heterocyclic group, and R⁴ is a hydrogen atom or a halogen; or a prodrug thereof or a pharmaceutically acceptable salt of them or a solvate of them; Even more preferably, the pharmaceutical composition of the present invention, in which the group V and/or X sPLA₂-inhibiting compound is shown by general formula (I) in which
   R⁵ is a group of -(CH₂)₁₋₆-R¹⁵ wherein R¹⁵ is a group of formula: 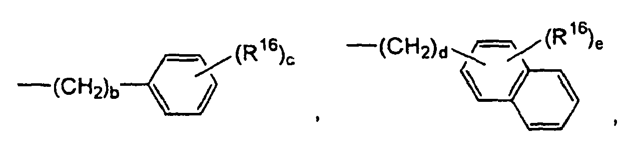 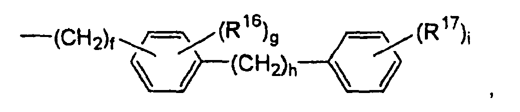 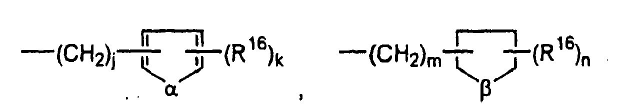 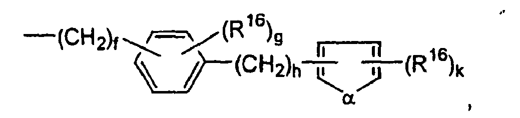 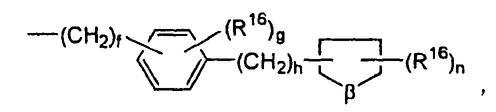 or 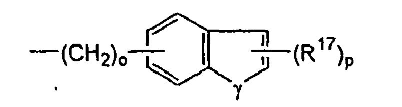 in which each b, d, f, h, j, m, and o is independently an integer of 0 to 2; each R¹⁶ and R¹⁷ is a group selected independently from a halogen, a C1-C10 alkyl, a C1-C10 alkyloxy, a C1-C10 alkylthio, an aryloxy, a phenyl, and a C1-C10 haloalkyl; α is an oxygen atom or a sulfur atom; β is -CH₂- or -(CH₂)₂-; γ is an oxygen atom or a sulfur atom; c, i, and p are an integer of 0 to 5; e is an integer of 0 to 7; g is an integer of 0 to 4; and each k and n is independently an integer of 0 to 3; or a prodrug thereof or a pharmaceutically acceptable salt of them or a solvate of them;
   Even much more preferably, the pharmaceutical composition of the present invention, in which the group V and/or X sPLA₂-inhibiting compound is shown by general formula (I) in which
   R⁵ is a group of -CH₂-R¹⁸ wherein R¹⁸ is a group of formula: 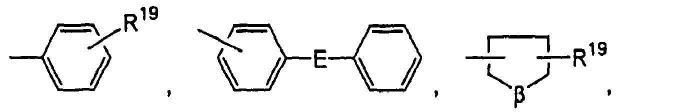 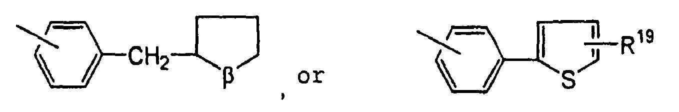 in which β is -CH₂- or -(CH₂)₂-; R¹⁹ is a hydrogen atom, a C1-C3 alkyl, or a halogen; and E is a single bond, -CH₂-, or -O-; or a prodrug thereof or a pharmaceutically acceptable salt of them or a solvate of them;
   Even more preferably, the pharmaceutical composition of the present invention, in which the group V and/or X sPLA₂-inhibiting compound is shown by general formula (I) in which R¹ is -OCH₂COOH; or a prodrug thereof or a pharmaceutically acceptable salt of them or a solvate of them;
   Even more preferably, the pharmaceutical composition of the present invention, in which the group V and/or X sPLA₂-inhibiting compound is shown by general formula (I) in which R² is a hydrogen atom; or a prodrug thereof or a pharmaceutically acceptable salt of them or a solvate of them;
   Even more preferably, the pharmaceutical composition of the present invention, in which the group V and/or X sPLA₂-inhibiting compound is shown by general formula (I) in which R⁶ is a C1-C3 alkyl; or a prodrug thereof or a pharmaceutically acceptable salt of them or a solvate of them;
   Even more preferably, the pharmaceutical composition of the present invention, in which the group V and/or X sPLA₂-inhibiting compound is shown by general formula (I) in which R^{A} is -CH₂CONH₂ or -COCONH₂; or a prodrug thereof or a pharmaceutically acceptable salt of them or a solvate of them;
   Even more preferably, the pharmaceutical composition of the present invention, in which the group V and/or X sPLA₂-inhibiting compound is shown by the formula: 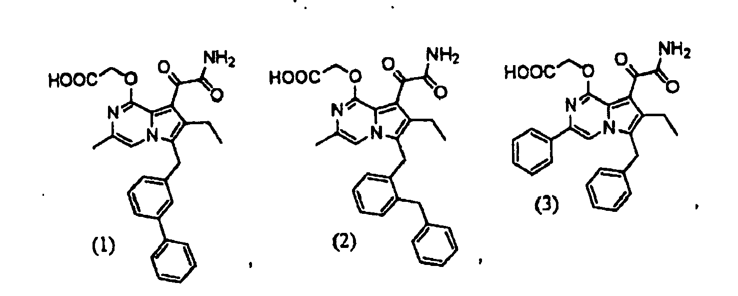 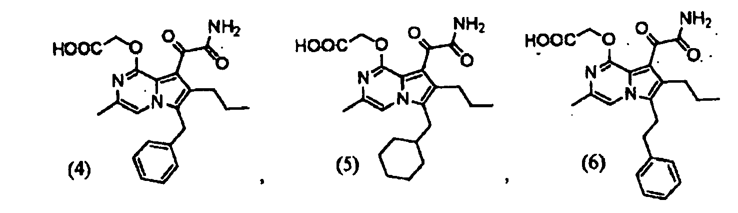 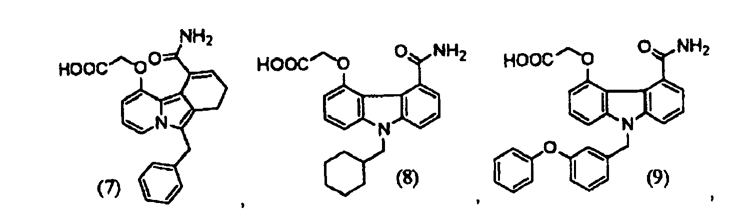 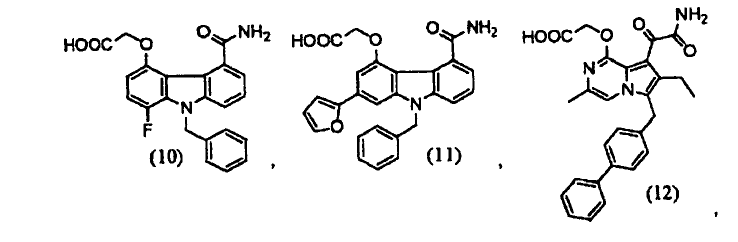 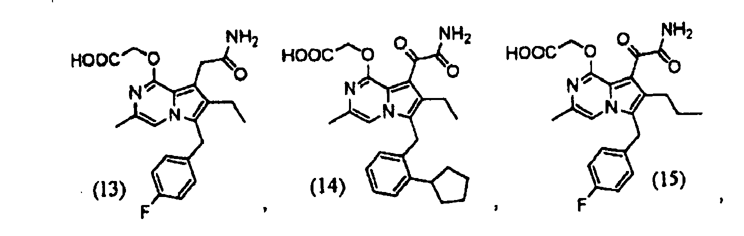 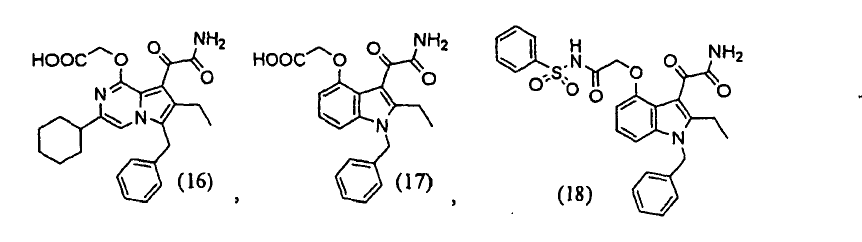 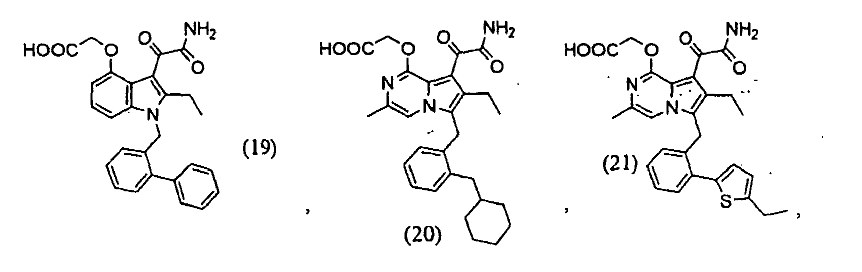 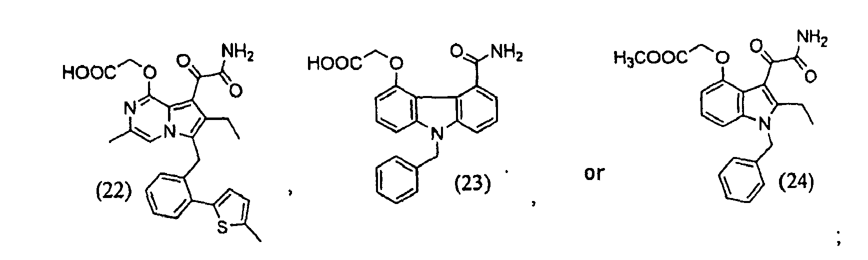 or a prodrug thereof or a pharmaceutically acceptable salt of them or a solvate of them;
   Specifically, the pharmaceutical composition of the present invention, in which the group V sPLA₂-inhibiting compound is shown by the formula: 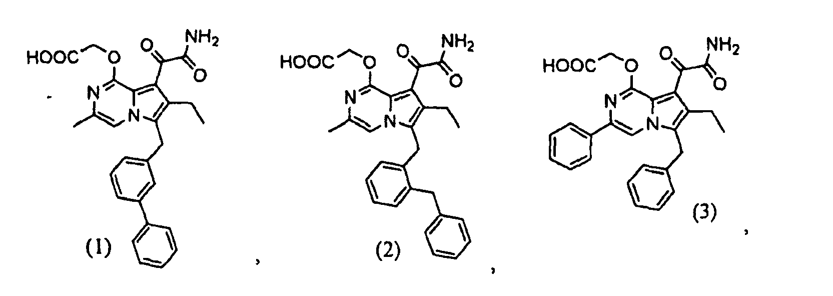 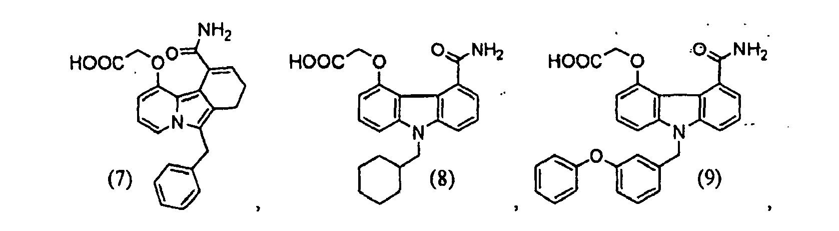 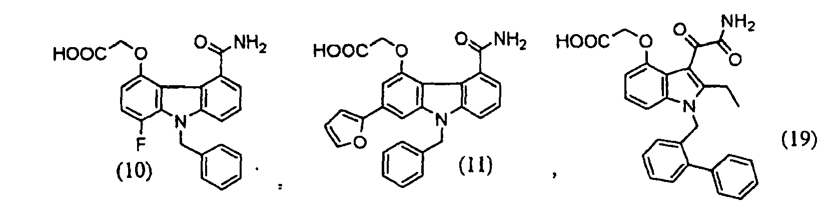 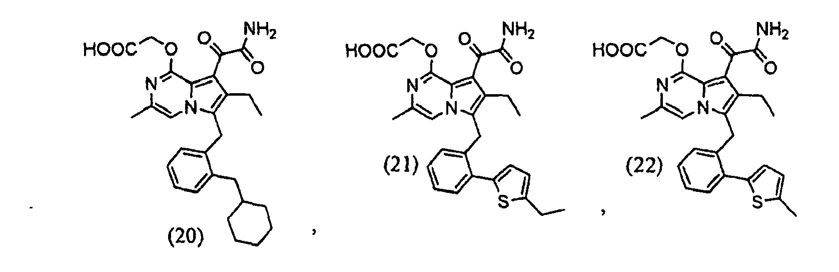 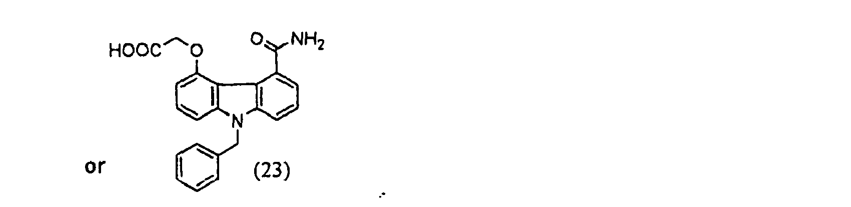 or a prodrug thereof or a pharmaceutically acceptable salt of them or a solvate of them;
   Alternatively, the pharmaceutical composition of the present invention, in which the group X sPLA₂-inhibiting compound is shown by the formula: 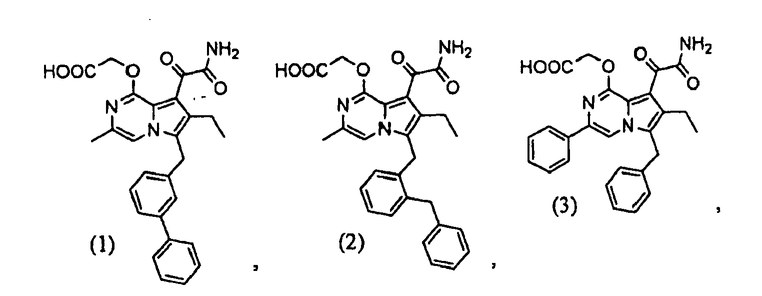 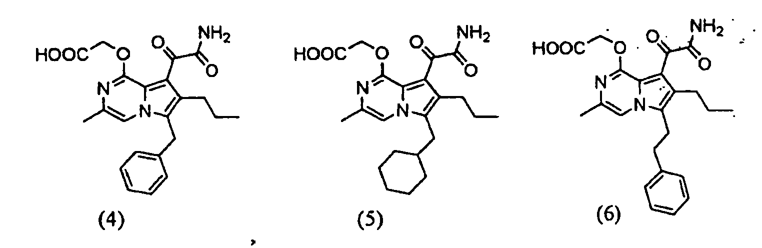 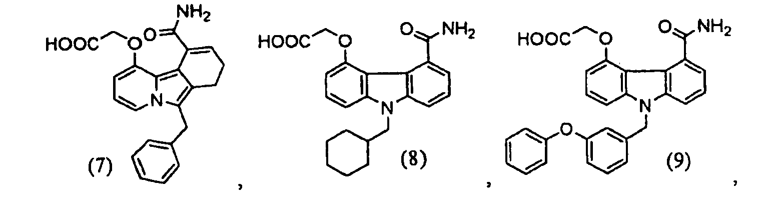 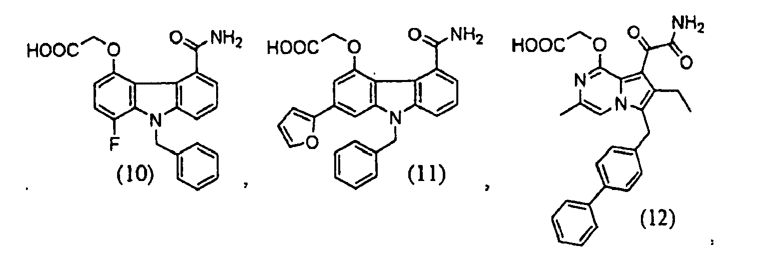 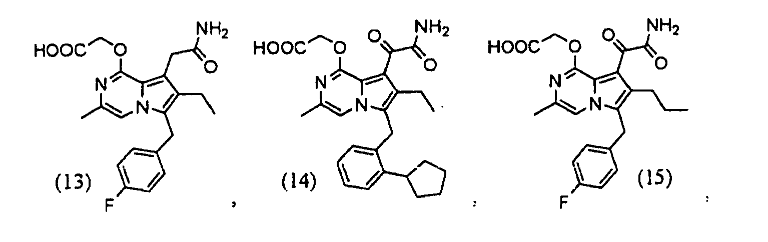 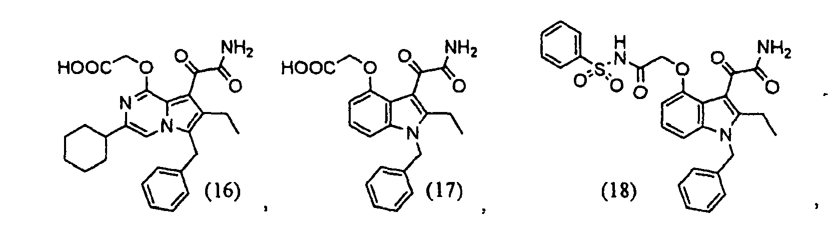 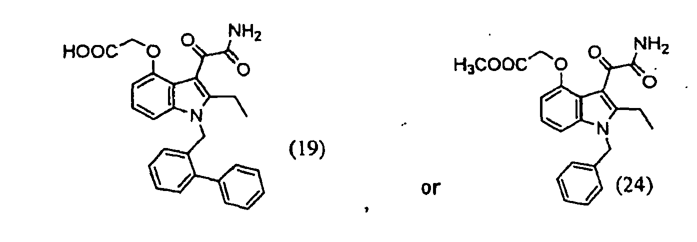 or a prodrug thereof or a pharmaceutically acceptable salt of them or a solvate of them.
(3) Use of a group V and/or X sPLA₂-inhibiting compound for preparation of a medicament for treating or preventing arteriosclerosis or an ischemic disease based on arteriosclerosis; preferably, the use of the present invention in which the group V and/or X sPLA₂-inhibiting compound is a compound as described above; and
(4) A method for treating or preventing arteriosclerosis or an ischemic disease based on arteriosclerosis in a mammal including a human, which comprises administrating to said mammal a therapeutically effective amount of a group V and/or X sPLA₂-inhibiting compound, thereby alleviating their symptoms; preferably, the method of the present invention, in which the group V and/or X sPLA₂-inhibiting compound is a compound as described above.

### BRIEF DESCRIPTION OF DRAWINGS

Fig. 1 represents a graph showing does-dependent release of arachidonic acid from LDL induced by group IB, IIA and X sPLA₂ s (at 37 °C for one hour).
Fig. 2 represents a graph showing effects of inhibitors on release of arachidonic acid from LDL induced by group X sPLA₂ (sPLA₂ inhibitor: indoxam, COX inhibitor: indomethacin, 5-LOX inhibitor: AA-861, each 10 µmol/L, the results are expressed as the percent of arachidonic acid released by group X sPLA₂ in the absence of inhibitors).
Fig. 3 represents a graph showing changes in the contents of a major phospholipid of LDL, phosphatidylcholine (PC) (Fig. 3A), and lyso-form thereof (lyso-PC) (Fig. 3B), when LDL is reacted with group IB, IIA and X sPLA₂ s, and CuSO₄ for 3, 6 and 24 hours.
Fig. 4 represents a photograph substitute for drawing which shows the results of analysis of agarose gel electrophoresis for electric charge of LDL subjected to the reactions with group IB, IIA and X sPLA₂ s, and CuSO₄ for 3, 6 and 24 hours (the upper: cathode, the lower: anode).
Fig. 5 represents a graph showing changes in peroxidation of lipid contained in LDL (A) and HDL (B) subjected to the reactions with group IB, IIA and X sPLA₂ s, and CuSO₄ for 3, 6 and 24 hours, in which the thiobarbituric acid reactive substances (TBARS) are expressed in terms of malondialdehyde (MDA).
Fig. 6 represents a graph showing reduction in cholesterol efflux induced by group X sPLA₂-degenerated HDL. Addition of non-degenerated HDL (natural HDL) to medium reduced significantly the level of intracellular cholesterol ester compared to the medium only, whereas addition of group X-degenerated HDL (X-HDL) significantly suppress the reduction.
Fig. 7 represents a graph showing time-dependent fatty acid release from LDL induced by group IIA or V sPLA₂ (at 37 °C, addition of 50 nmol/L sPLA₂).
Fig. 8 represents a graph showing effects of inhibitors on release of linoleic acid from LDL induced by group V sPLA₂ (sPLA₂ inhibitor: indoxam, COX inhibitor: indomethacin, each 10 µM, the results are expressed as the percent of linoleic acid released by group V sPLA₂ in the absence of inhibitors).
Fig. 9 represents a graph showing changes in the contents of a major phospholipid of LDL, phosphatidylcholine (PC) (A), and lyso-form thereof (lyso-PC) (B), when LDL is reacted with group IIA and V sPLA₂ s, or CuSO₂ for 3, 6 and 24 hours (at 37 °C, addition of 50 nmol/L sPLA₂).
Fig. 10 represents a photograph substitute for drawing which shows the results of analysis of agarose gel electrophoresis for electric charge of LDL subjected to the reactions with group V sPLA₂ for 3, 6 and 24 hours (the upper: cathode, the lower: anode).

### BEST MODE FOR CARRYING OUT THE INVENTION

### (1) A pharmaceutical composition for suppressing degeneration of serum lipoproteins, which comprises a group V and/or X sPLA₂-inhibiting compound as an active ingredient

The inventors of the present invention found that human group V and X sPLA₂s powerfully releases fatty acids from the phospholipid of lipoproteins in blood. Further, the inventors showed that group V and/or X sPLA₂ is very potent in degeneration of LDL and HDL, including phospholipolysis, release of fatty acids, production of lysophospholipids, increase in negative charge, and uptake into macrophage, by comparing with those of group IB and group IIA sPLA₂, and found that these actions of group V and X sPLA₂s differ from degeneration actions of lipoproteins caused by oxidation, which have been previously reported. In this context, the term "degeneration of serum lipoproteins" according to the present invention refers to phospholipolysis, release of fatty acids, production of lysophospholipid, increase of negative charge, uptake into macrophage and the like, and does not include degeneration by oxidation.

"A group V and/or X sPLA₂-inhibiting compound" or "a group V and/or X sPLA₂-inhibitor" as used in the invention includes a compound that inhibits group V sPLA₂, a compound that inhibits group X sPLA₂, and a compound that has an inhibitory activity on both group V and X sPLA₂s. In an embodiment, the present invention encompasses a pharmaceutical composition that comprises both a compound that inhibits group V sPLA₂ and a compound that inhibits group X sPLA₂ as active ingredients.

The inventors also found that group X sPLA₂ is expressed in foam cells at vascular lesions in a pathologic animal model of arteriosclerosis by employing an antibody specific to group X sPLA₂. Thus, the present invention encompasses a pharmaceutical composition for treating or preventing arteriosclerosis or for treating or preventing an ischemic disease based on arteriosclerosis, which comprises a group V and/or X sPLA₂-inhibiting compound as an active ingredient. The term "arteriosclerosis" as used herein is a general term that means a pathology wherein the artery wall thickens and hardens, and refers to any pathological embodiment that leads to cerebral ischemic attack, cerebral infarction, angina pectoris, myocardial infarction, and intermittent claudication, all of which are caused by constriction and occlusion of artery. According to the present invention, atherosclerosis among the arteriosclerosis is preferably treated. The term "ischemic disease based on arteriosclerosis" as used herein refers to an ischemic disease caused by or associated with arteriosclerosis. Ischemic diseases in general include various clinical pathological embodiments caused by topical anemia, such as necrosis. However, in accordance with the present invention, the term "ischemic disease" refers to an ischemic cardiac disease caused by occlusive ischemia due to alterations in vessel or of vessel itself, such as angina pectoris, myocardial infarction, cardiac failure, and cerebral infarction.

### 2) The pharmaceutical composition of the present invention, which comprises a group V and/or X sPLA₂-inhibiting compound shown by general formula (I)

The term "alkyl" as used in formula (I) solely or in combination with other terms means a straight or branched monovalent hydrocarbon radial, which has an indicated number of carbon atom. The radicals include, for example, methyl, ethyl, n-propyl, isopropyl, n-butyl, isobutyl, sec-buthyl, tert-butyl, n-pentyl, n-hexyl, n-heptyl, n-octyl, n-nonyl, n-decanyl, n-undecanyl, n-dodecanyl, n-tri-decanyl, n-tetra-decanyl, n-penta-decanyl, n-hexa-decanyl, n-hepta-decanyl, n-octa-decanyl, n-nona-decanyl, n-eicosanyl, and the like.

The term "alkenyl" as used in formula (I) solely or in combination with other terms means a straight or branched monovalent hydrocarbon radial, which has an indicated number of carbon atom, and have one or more double bonds. The radicals include, for example, vinyl, allyl, propenyl, crotonyl, isopentenyl, various butenyl isomers, and the like.

The term "alkynyl" as used in formula (I) solely or in combination with other terms means a straight or branched monovalent hydrocarbon radial, which has an indicated number of carbon atom, and have one or more triple bonds. The radicals include, for example, ethynyl, propynyl, 6-heptynyl, 7-octynyl, 8-nonyl, and the like.

The term "carbocyclic group" as used in formula (I) means a radical derived from a saturated or unsaturated, substituted or unsubstituted 5 to 14-, preferably 5 to 10-, more preferably 5 to 7-membered organic nucleus whose ring forming atoms (other than hydrogen) are solely carbon atoms. The term includes a group wherein two or three of the radial as mentioned above are connected each other. Typical carbocyclic groups include cycloalkyls such as cyclopropyl, cyclobutyl, cyclopentyl, cyclohexyl, cycloheptyl and cyclooctyl; cycloalkenyls such as cyclobutenyl, cyclopentenyl, cyclohexenyl, cycloheptenyl and cyclooctenyl, phenyl, naphthyl, norbornanyl, bicycloheptadienyl, indenyl, stilbenyl, terphenylyl, phenyl-cyclohexenyl, acenaphthyl, anthryl, biphenylyl, bibenzylyl and phenylalkylphenyl derivatives shown by formula (II):

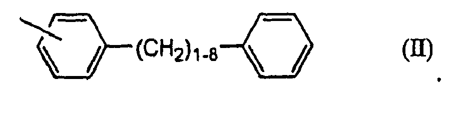

Preferred carbocyclic group in R³ and R⁴ includes phenyl, cyclohexyl, and the like.

The term "heterocyclic group" as used in formula (I) means a radical derived from monocyclic or polycyclic, saturated or unsaturated, substituted or unsubstituted heterocyclic nuclei having 5 to 14 ring atoms and containing from 1 to 3 hetero atoms selected from the group consisting of nitrogen, oxygen and sulfur. Heterocyclic groups include, for example, pyridyl, pyrrolyl, furanyl, benzofuranyl, thienyl, benzothienyl, pyrazolyl, imidazolyl, phenylimidazolyl, triazolyl, isoxazolyl, oxazolyl, thiazolyl, thiadiazolyl, indolyl, carbazolyl, norharmanyl, azaindolyl, benzofuranyl, dibenzofuranyl, dibenzothiophenyl, indazolyl, imidazo[1.2-a]pyridinyl, benzotriazolyl, anthranilyl, 1,2-benzisoxazolyl, benzoxazolyl, benzothiazolyl, purinyl, pyridinyl, dipyridinyl, phenylpyridinyl, benzylpyridinyl, pyrimidinyl, phenylpyrimidinyl, pyrazinyl, 1,3,5-triazinyl, quinolinyl, phthalazinyl, quinazolinyl, quinoxalinyl, and the like.

Preferred heterocyclic group in R³ and R⁴ includes furyl, thienyl, and the like.

Preferred carbocyclic group and heterocyclic group in group R⁵ includes a radial shown by the formula: 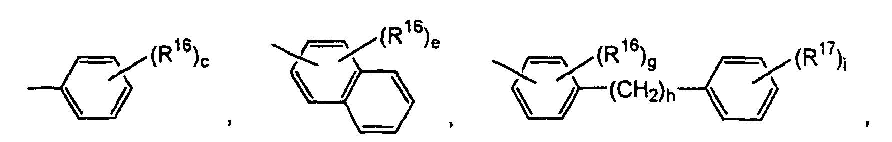 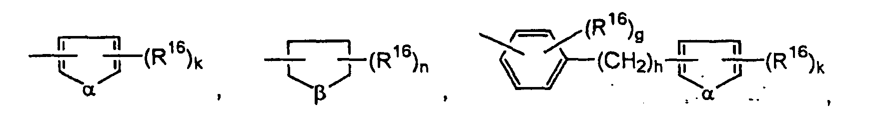 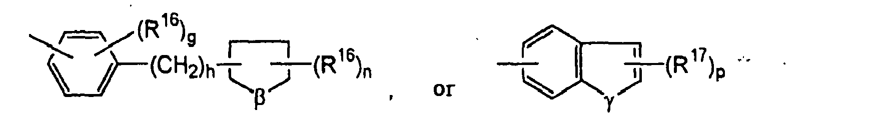 in which h is an integer of 0 to 2; each R¹⁶ and R¹⁷ is a group selected independently from a halogen, a C1-C10 alkyl, a C1-C10 alkyloxy, a C1-C10 alkylthio, an aryloxy, a phenyl, and a C1-C10 haloalkyl; α is an oxygen atom or a sulfur atom; β is -CH₂- or -(CH₂)₂-; γ is an oxygen atom or a sulfur atom; c, i, and p are an integer of 0 to 5; e is an integer of 0 to 7; g is an integer of 0 to 4; and each k and n is independently an integer of 0 to 3. When c, e, g, i, k, n, and/or p is 2 or more, then each one of groups R¹⁶ and groups R¹⁷ may be different each other. When R¹⁶ is a substituent on a naphthyl group, R¹⁶ may be substituted at any position on the naphthyl group.

More preferable groups include a radial shown by the formula: 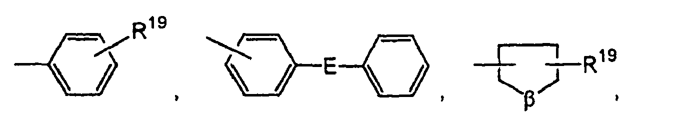 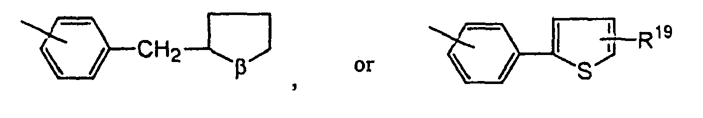 in which R¹⁹ is a hydrogen atom, a C1-C3 alkyl, or a halogen; E is a single bond, -CH₂- or -O-; β is -CH₂- or -(CH₂)₂-.

Preferred group of R⁵ includes a "carbocyclic group"-C1-C3 alkyl, and a "heterocyclic group"-C1-C3 alkyl.

The term "non-interfering substituent" as used in formula (I) means a radical suitable for substitution on "carbocyclic group", "heterocyclic group" as defined above, and the basic nucleus. Exemplified non-interfering radicals include a C1-C10 alkyl, a C2-C6 alkenyl, a C2-C6 alkynyl, a C7-C12 aralkyl (such as benzyl and phenethyl), a C7-C12 alkaryl, a C3-C8 cycloalkyl, a C3-C8 cycloalkenyl, phenyl, tolyl, xylyl, biphenyl, a C1-C10 alkyloxy, a C1-C6 alkyloxy-C1-C6 alkyl (such as methyloxymethyl ethyloxymethyl, methyloxyethyl, and ethyloxyethyl), a C1-C6 alkyloxy-C1-C6 alkyloxy (such as methyloxymethyloxy and methyloxyethyloxy), a C 1-C6 alkylcarbonyl (such as methylcarbonyl and ethylcarbonyl), a C 1-C6 alkylcarbonylamino (such as methylcarbonyamino and ethylcarbonylamino), a C1-C6 alkyloxyamino (such as methyloxyamino and ethyloxyamino), a C1-C6 alkyloxyaminocarbonyl (such as methyloxyaminocarbonyl and ethyloxyaminocarbonyl), a mono- or di-C1-C6 alkylamino (such as methylamino, ethylamino, dimethylamino, and ethylmethylamino), a C1-C10 alkylthio, a C1-C6 alkylthiocarbonyl (such as methylthiocarbonyl and ethylthiocarbonyl), a C1-C6 alkylsulfinyl (such as methylsulfinyl and ethylsulfinyl), a C1-C6 alkylsulfonyl (such as methylsulfonyl and ethylsulfonyl), a C2-C6 haloalkyloxy (such as 2-chloroethyloxy and 2-bromooethyloxy), a C1-C6 haloalkylsulfonyl (such as chloromethylsulfonyl and bromomethylsulfonyl), a C1-C10 haloalkyl, a C1-C6 hydroxyalkyl (such as hydroxymethyl and hydroxyethyl), a C1-C6 alkyloxycarbonyl (such as methyloxycarbony and ethyloxycarbonyl), -(CH₂)₁₋₈-O-(a C1-C6 alkyl), benzyloxy, an aryloxy (such as phenyloxy), an arylthio (such as phenylthio), -(CONHSO₂ R²⁰ wherein R²⁰ is a C1-C6 alkyl or an aryl), - CHO, amino, amidino, a halogen, carbamyl, carboxyl, carbalkyloxy, - (CH₂)₁₋₈-COOH (such as carboxymethyl, carboxyethyl and carboxypropyl), cyano, cyanoguanidino, guanidino, hydrazido, hydrazino, hydroxy, hydroxyamino, nitro, phosphono, -SO₃H, thioacetal, thiocarbonyl, a C1-C6 carbonyl, a carbocyclic group, a heterocyclic group, and the like. These substituents may be substituted by one or more substituent(s) selected from a group consisting of a C1-C6 alkyl, a C1-C6 alkyloxy, a C2-C6 haloalkyloxy, a C1-C6 haloalkyl, and a halogen.

Preferred non-interfering substituent as used as "substituted by a non-interfering substituent" in R³, R⁴ and R⁵ includes a halogen, a C1-C6 alkyl, a C1-C6 alkyloxy, a C1-C6 alkylthio, and a C1-C6 haloalkyl. More preferred one includes a halogen, a C1-C3 alkyl, a Cl-C3 alkyloxy, a C1-C3 alkylthio, and a C1-C3 haloalkyl.

Preferred non-interfering substituent as used in R¹ , R² , R³ , R⁴ , and R⁷ includes a C1-C6 alkyl, an aralkyl, a C1-C6 alkyloxy, a C1-C6 alkylthio, a C1-C6 hydroxyalkyl, a C2-C6 haloalkyloxy, a halogen, a carboxy, a C1-C6 alkyloxycarbonyl, an aryloxy, an arylthiol, a carbocyclic group, and a heterocyclic group. More preferred one includes a C1-C6 alkyl, an aralkyl, a carboxy, a C1-C6 hydroxyalkyl, phenyl, and a C1-C6 alkyloxycarbonyl.

The term "halogen" as used in formula (I) means fluoro, chloro, bromo, or iodo.

The term "cycloalkyl" as used in formula (I) means a monovalent cyclic hydrocarbon radial having an indicated number of carbon atom. The radicals include, for example, cyclopropyl, cyclobutyl, cyclopentyl, cyclohexyl, cycloheptyl, cyclooctyl, and the like.

The term "cycloalkenyl" as used in formula (I) means a monovalent cyclic hydrocarbon radial having an indicated number of carbon atom, and containing one or more double bond(s). The radicals include, for example, 1-cyclopropenyl, 2-cyclopropenyl, 1-cyclobutenyl, 2-cyclobutenyl, and the like.

The term "alkyloxy" as used in formula (I) include for example methyloxy, ethyloxy, n-propyloxy, isopropyloxy, n-butyloxy, n-pentyloxy, n-hexyloxy, and the like.

The term "alkylthio" as used in formula (I) include for example methylthio, ethylthio, n-propylthio, isopropylthio, n-butylthio, n-pentylthio, n-hexylthio, and the like.

The term "acidic group" as used in formula (I) means an organic group which when attached to a nucleus through suitable linking atoms (hereinafter defined as the "a linker group to the acidic group"), acts as a proton donor capable of hydrogen bonding. Acidic groups include, for example, a group shown by the formula: 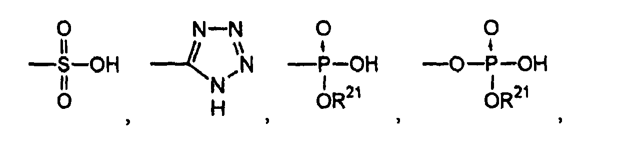 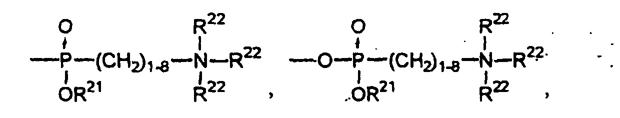 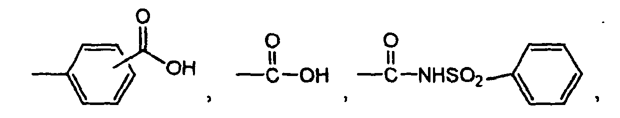 or 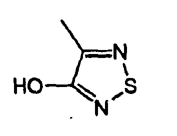 in which R²¹ is a hydrogen atom, a metal, or a C1-C10 alkyl, and each R²² is independently a hydrogen atom or a C1-C10 alkyl, provided that, when an acidic group has both R²¹ and R²², then at least one of R²¹ and R²² is a hydrogen atom. Preferred group includes -COOH, -SO₃H, - CONHSO₂ C₆ H₅, or P(O)(OH)₂, and more preferred one includes - COOH.

The term "a linker group to the acidic group" as used in formula (I) means a divalent linking group symbolized as, -(L¹)-, which has the function of joining the nucleus to an acidic group in the general relationship. The group includes, for example, a group shown by the formula: 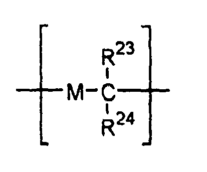 in which M is -CH₂ -, -O-, -N (R²⁵)-, or -S-, and each R²³ and R²⁴ is independently a hydrogen atom, a C1-C10 alkyl, an aryl, an aralkyl, a carboxy, or a halogen, and a group shown by the formula: 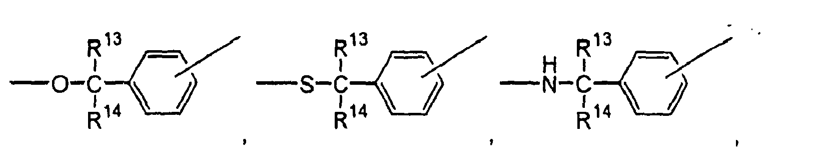 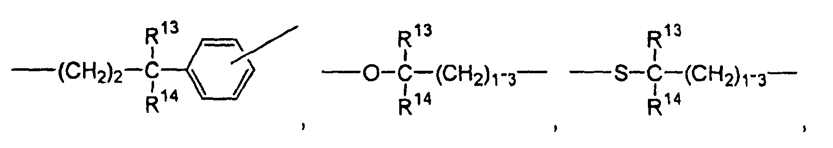 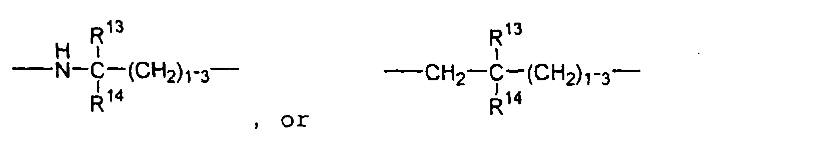 in which each R¹³ and R¹⁴ is independently a hydrogen atom, a C1-C10 alkyl, a C1-C10 aralkyl, a carboxy, an alkyloxycarbonyl, or a halogen. Preferred one includes, for example, -O-CH₂-, -S-CH₂-, -N (R²⁵)-CH₂-,-CH₂ -CH₂-, -O-CH (CH₃)-, and -O-CH((CH₂)₂C₆H₅)- wherein R²⁵ is a C1-C6 alkyl, and more preferred one includes -O-CH₂- and -S-CH₂-.

The term "the length of the linker to the acidic group" as used in formula (I) means the number of atoms (excluding hydrogen) in the shortest chain of the linker group -(L¹)- that connects the nucleus with the acidic group. The presence of a carbocyclic ring in -(L¹)- counts as the number of atoms approximately equivalent to the calculated diameter of the carbocyclic ring. Thus, a benzene or cyclohexane ring in the linker to the acid group counts as 2 atoms in calculating the length of -(L¹)-. Preferred length is 2 to 3.

The term "haloalkyl" as used in formula (I) means "an alkyl" as defined above substituted at any position with a halogen as defined above. Haloalkyl includes, for example, chloromethyl, trifluoromethyl, 2-chloromethyl, 2-bromomethyl and the like.

The term "hydroxyalkyl" as used in formula (I) means "an alkyl" as defined above substituted at any position with hydroxy. Hydroxyalkyl includes, for example, hydroxymethyl, 2-hydroxyethyl, 3-hydroxypropyl, and the like, with hydroxymethyl being preferred.

The term "haloalkyl" in "haloalkyloxy" as used in formula (I) is as defined above. Haloalkyloxy includes, for example, 2-chloroethyloxy, 2-trifluoroethyloxy, 2-chloroethyloxy, and the like.

The term "aryl" as used in formula (I) means a monocyclic or condensed aromatic hydrocarbon group, and includes, for example, phenyl, 1-naphthyl, 2-naphthyl, anthryl, and the like, with phenyl or 1-naphthyl being preferred.

The term "aralkyl" as used in formula (I) means "an alkyl" as defined above substituted at any substituable position with "an aryl" as defined above, and includes, for example, benzyl, phenethyl, phenylpropyl (e.g. 3-phenylpropyl), naphthylmethyl (e.g. 1-naphthylmethyl) and the like.

The term "alkyloxycarbonyl" as used in formula (I) includes, for example, methyloxycarbony, ethyloxycarbonyl, n-propyloxycarbonyl, and the like.

The term "aryloxy" as used in formula (I) includes, for example, phenyloxy and the like.

The term "arylthio" as used in formula (I) includes, for example, phenylthio and the like.

The term "halophenyl" as used in formula (I) means a phenyl substituted at one or more position(s) with "a halogen" as defined above, and includes, for example, fluorophenyl, chlorophenyl, bromophenyl, iodophenyl, difluorophenyl, dichlorophenyl, dibromophenyl, trifluorophenyl, trichlorophenyl, tribromophenyl, chlorofluorophenyl, bromochlorophenyl, and the like.

"Cyclohexene ring" in ring D as used in formula (I) means a cyclohexen ring having, in the ring, only one double bond at the condensed part sharing with the adjacent ring.

Preferred combination between ring A and -B- are one of (m) to (r) in the followings: 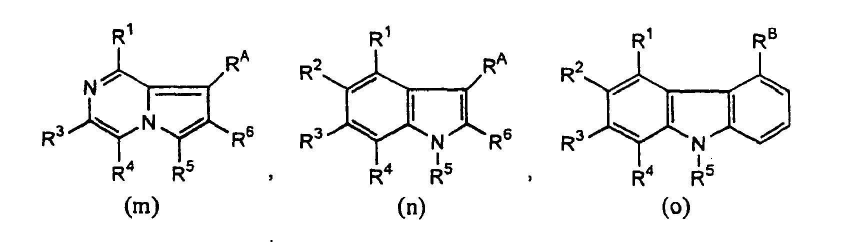 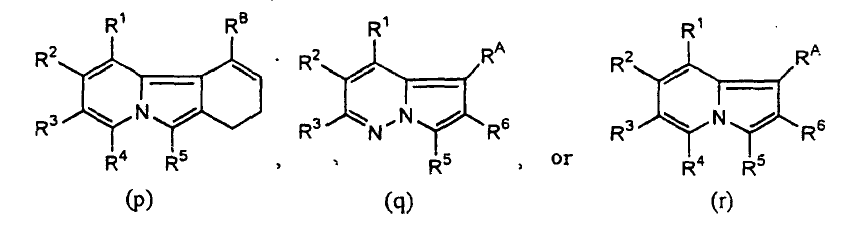 with one of (m) to (p) being more preferred.

Group V and/or X sPLA₂-inhibiting compounds shown by general formula (I) can be prepared in accordance with the well-known preparation described in EP-620214 (Japanese Patent Publication (kokai) No. 7-010838, US-5578634), EP-620215 (Japanese Patent Publication (kokai) No. 7-025850, US-5684034), EP-675110 (Japanese Patent Publication (kokai) No. 7-285933, US-5654326), WO96/03120 (Japanese Patent Publication (kokai) No. 10-505336), WO96/03383 (Japanese Patent Publication (kokai) No. 10-505584), W098/ 18464 (EP839806), W099 / 51605, W099 / 59999, or the like.

Group X sPLA₂-inhibiting compounds can be selected by preparing cells that express human group X sPLA₂ and the culture supernatant therefor, and then assaying candidates for the inhibitory activity as described hereinafter. Specifically, the cDNA sequence encoding human group X sPLA₂ (Cupillard, et al., J. Biol. Chem, 1997, 272, 15745-15752) is inserted into an expression vector for animal cells. The resultant expression vector is transfected into a host cell to provide a cell that stably expresses human group X sPLA₂. The cells are cultured to prepare the culture supernatants therefor. Then, chromogenic assay as described below is used to identify group X sPLA₂-inhibiting compounds and estimate the activity. The general guidance of this assay is described in the article "Analysis of Human Synovial Fluid Phospholipase A2 on Short Chain Phosphatidylcholine-Mixed Micelles: Development of a Spectrophotometric Assay Suitable for a Microtiterplate Reader", Analytical Biochemistry, 204, pp 190-197,1992 by Laure J. Reynolds, Lori L. Hughes and Edward A. Dennis.

Group V sPLA₂-inhibiting compounds can be selected by a similar procedure to that described above except for the use of the cDNA sequence encoding human group V sPLA₂ (Chen, et al., J. Biol. Chem, 1994, 269, 2365-2368).

When a group V and/or X sPLA₂-inhibiting compound has an acidic group or a basic group, it can be formed to a salt which is more soluble in water and is more physiologically suitable. Typical pharmaceutically acceptable salts include, but not limited to, salts formed with an alkali metal or an alkaline-earth metal such as lithium sodium, potassium, calcium, magnesium, aluminum, or the like. Salts can be readily made from a free acid by treating the acid in a solution with a base, or contacting the acid to an ion-exchange resin. The pharmaceutically acceptable salts include addition salts made from a relatively nontoxic inorganic and organic base of a compound for treatment or prevention of ischemic reperfusion injury, such as an amine cation, an ammonium, a quaternary ammonium derived from a nitrogen base that are basic sufficiently to form a salt with the compound (for example, S.M.Berge, et al., "Pharmaceutical Salts," J.Phar.Sci., 66, 1-19 (1977)). Further, the basic group contained in the group V and/or X sPLA₂-inhibiting compound can be reacted with a suitable organic or inorganic acid to form salts such as acetates, benzenesulfonates, benzoates, bicarbonates, bisulfonates, bitartrates, borates, bromides, camsylates, carbonates, chlorides, clavulanates, citrates, edetates, edisylates, estolates, esylates, fluorides, fumarates, gluceptates, gluconates, glutamates, glycolylarsanilates, hexylresorcinates, hydroxynaphthoates, iodide, isothionates, lactates, lactobionates, laurates, malates, malseates, mandelates, mesylates, methylbromides, methylnitrates, methylsulfates, mucates, napsylates, nitrates, oleates, oxalates, palmitates, pantothenates, phosphates, polygalacturonates, salicylates, stearates, subacetates, succinates, tannates, tartrates, tosylates, trifluoroacetates, trifluoromethane sulfonates, valerates, and the like. Solvates as used hrein include those formed with an organic solvent and/or water. Hydrates may be formed with any number of water molecule.

When a group V and/or X sPLA₂-inhibiting compound has one or more chiral centers, those may exist in optically active forms. Likewise, when the compounds contain an alkenyl or alkenylene group, there exists the possibility of cis- and trans- isomeric forms of the compounds. The R- and S- isomers and mixtures thereof, including racemic mixtures as well as mixtures of cis- and trans- isomers, are contemplated by this invention. Additional asymmetric carbon atoms can be present in a substituent group such as an alkyl group. All such isomers as well as the mixtures thereof are intended to be included in the invention. If a particular stereoisomer is desired, it can be prepared by methods well-known in the art by using stereospecific reactions with starting materials which contain the asymmetric centers and are already resolved or, alternatively by methods which lead to mixtures of the stereoisomers and subsequent resolution by known methods.

Prodrugs as used herein mean derivatives of the group V and/or X sPLA₂-inhibiting compounds, which have chemically or metabolically cleavable groups and become by solvolysis or under physiological conditions the compounds which are pharmaceutically active in vivo. The prodrug derivatives are active in both acid and base derivatives, and the acid derivatives offers advantages of solubility, tissue compatibility, or release control in a mammalian organism (Bundgard, H., Design of Prodrugs, pp. 7-9, 21-24, Elsevier, Amsterdam 1985). For example, prodrugs including acid derivatives are well known in the art, such as esters prepared by reaction of the parent acidic compound with a suitable alcohol, or amides prepared by reaction of the parent acid compound with a suitable amine. Simple aliphatic or aromatic esters derived from acidic groups pendent on the compounds are preferred prodrugs. More preferable includes C1-C6 alkyl esters of acidic groups, such as methyl ester, ethyl ester. In some cases it is possible to prepare double ester type prodrugs such as (acyloxy) alkyl esters or ((alkoxycarbonyl)oxy)alkyl esters.

The term "pharmaceutically acceptable" means carriers, diluents or additives that are compatible with other ingredients in the composition and harmless to a recipient.

The compositions for treatment or prevention according to the present invention can be administered by a variety of routes including oral, aerosol, rectal, transdermal, subcutaneous, intravenous, intramuscular, and intranasal. Formulations of the invention are prepared by combining (e.g., mixing) a therapeutically effective amount of the compounds together with a pharmaceutically acceptable carrier or diluent therefor. The formulations are prepared by known procedures using well-known and readily available ingredients.

In making the compositions of the present invention, the active ingredient is admixed with a carrier, or diluted by a carrier, or enclosed within a carrier which may be in the form of a capsule, sachet, paper or other container. When the carrier serves as a diluent, it may be a solid, semi-solid or liquid material which acts as a medium, or can be in the form of tablets, pills, powders, lozenges, elixirs, suspensions, emulsions, solutions, syrups, aerosols (as a solid in a liquid medium), or ointment, containing, for example, up to 10% of the active compound. The compounds exhibiting an anti-cancer activity of the present invention are preferably formulated prior to administration.

For the formulations, any suitable carrier known in the art can be used. In such a formulation, the carrier may be a solid, liquid, or mixture of a solid and a liquid. For instance, a compound for treatment or prevention of ischemic reperfusion injury is dissolved in a 4 % dextrose/0.5 % sodium citrate aqueous solution so as to be 2 mg/ml concentration for intravenous injection. Solid form formulations include powders, tablets and capsules. A solid carrier can be one or more substances which may also act as flavoring agents, lubricants, solubilisers, suspending agents, binders, tablet disintegrating agents and encapsulating material. Tablets for oral administration may contain suitable excipients such as calcium carbonate, sodium carbonate, lactose, calcium phosphate, together with disintegrating agents such as maize starch, or alginic acid, and/or binding agents such as gelatin or acacia, and lubricating agents such as magnesium stearate, stearic acid, or talc.

In powders, the carrier is a finely pulverized solid which is in admixture with the finely pulverized active ingredient. In tablets, the active ingredient is mixed with a carrier having the necessary binding properties in suitable proportions and compacted in the shape and size desired. The powders and the tablets preferably contain from about 1 to about 99 weight percent of the active ingredient which is the novel compound of this invention. Suitable solid carriers are magnesium carbonate, magnesium stearate, talc, sugar, lactose, pectin, dextrin, starch, gelatin, tragacanth, methyl cellulose, sodium carboxymethyl cellulose, low melting waxes, and cocoa butter.

Sterile liquid form formulations include suspensions, emulsions, syrups and elixirs. The active ingredient can be dissolved or suspended in a pharmaceutically acceptable carrier, such as sterile water, sterile organic solvent or a mixture of both. The active ingredient can often be dissolved in a suitable organic solvent, for example, aqueous propylene glycol. Other compositions can be made by dispersing the finely divided active ingredient in aqueous starch or sodium carboxymethyl cellulose solution or in a suitable oil.

Although an appropriate dosage varies depending on kind of disease, the administration route, age, body weight of the patient, in the case of intravenous administration, the dosage for an adult can be generally 0.01 to 10 mg/kg/hour, and preferably 0.1-1 mg/kg/hour.

### EXAMPLE

The following Examples are mere illustration of the present invention, but not intended to limit the scope of the invention.

### Example 1 Effect of group X sPLA₂ on fatty acid release from isolated human lipoproteins

LDL and HDL were isolated from human plasma by ultracentrifugation (Havel, et al., J. Clin. Invest. 34, 1345-1353 (1955)). Human group IB, IIA and X sPLA₂ s (0.5 nmol/L to 500 nmol/L) were reacted with LDL and HDL (0.35-1 mg/ml) at 37°C in a solution containing 12.5 mmol/L Tris-HCL buffer (pH 8.0), 125 mg/L bovine serum albumin, and 1 mmol/L calcium chloride. The fatty acids released from the lipoproteins were extracted according to the method of Dole (Dole, et al., J. Biol. Chem. 235, 2595-2599 (1960)), and were labeled with 9-anthryldiazomethane by the known method (Hanasaki, et al., J. Biol. Chem. 274, 34203-34211 (1999)). Then, the released fatty acids were determined by detecting fluorescence of the labeled products (fatty acids) eluted from high performance liquid chromatography (HPLC) on reverse-phase column (LichroCART 125-4 Superspher 100 RP-18 columnmol/Merck). In order to examine does-dependent release of fatty acids induced by each sPLA₂, the lipoprotein was reacted with 0.5, 5, 50, and 500 nmol/L group X sPLA₂, and 50 and 500 nmol/L group IB and IIA sPLA₂ for 60 minutes. Further, in order to examine time-dependent fatty acid release induced by sPLA₂, each 50 nmol/L sPLA₂ was used, and the change in the release with time course was traced during the period time of 4 hours after the sPLA₂ addition. In the experiment for determining the inhibitory activity of a sPLA₂ inhibitor (indoxam; Yokota, et al., Biochim. Biophys. Acta 1438, 213-222 (1999)), a cyclooxygenase (COX) inhibitor (indomethacin) or a 5-lipoxygenase (5-LOX) inhibitor (AA-861; 2,3,5-trimethyl-6-(12-hydroxy-5, 10- dodecadiynyl)-1, 4-benzoquinone (Yoshimoto, et al., Biochem. Biophys. Acta. 713, 470-473 (1982)), those agents were added to the reaction giving a final concentration of 10 µmol/L, virtually simultaneously with the addition of sPLA₂.

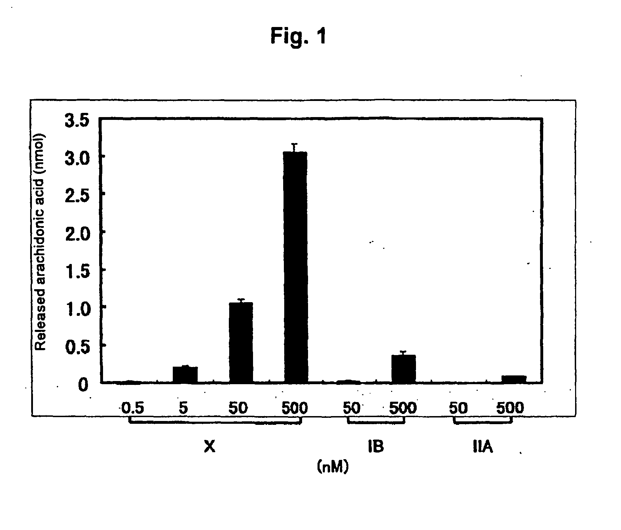

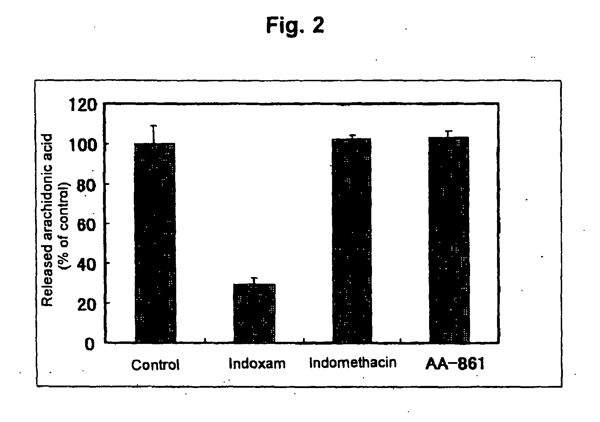

The experiment for time-dependent release revealed that only group X sPLA₂ among groups IB, IIA, and X sPLA₂ s released the fatty acids significantly. The fatty acid release from HDL induced by group X sPLA₂ reached a plateau at 60 minutes after the addition, whereas the fatty acid release from LDL continued to time-dependently increase for 4 hours. The experiment for does-dependent release of fatty acids showed that group X sPLA₂ at as low as 5 nmol/L significantly released fatty acids from LDL and HDL in a dose-dependent manner, whereas group IB and IIA sPLA₂ released only minor amounts of fatty acids even at a high dose of 500 nmol/L (Fig. 1). The fatty acid release induced by group X sPLA₂ was inhibited by a sPLA₂ inhibitor (indoxam), and it was affected neither by a COX inhibitor (indomethacin) nor a 5-LOX inhibitor (AA-861) (Fig. 2).

### Example 2 Effect of group X sPLA₂ on the phospholipid composition of lipoproteins

LDL and HDL were isolated from human plasma by ultracentrifugation (Havel, et al., J. Clin. Invest. 34, 1345-1353 (1955)). Human group IB, IIA and X sPLA₂s (50 nmol/L) and CuSO₄ (20 µmol/L) were reacted with LDL and HDL (1 mg/ml) at 37°C for 3, 6, and 24 hours in a solution containing 12.5 mmol/L Tris-HCL buffer (pH 8.0), 125 mg/L bovine serum albumin, and 1 mmol/L calcium chloride. Then, the change in the phospholipid composition of the LDL and HDL was traced by HPLC. Phospholipids were extracted from a 10 µg sample of LDL and a 15 µg sample of HDL according to the known method (Bligh, et al., Can. J. Biochem. Physiol. 37, 911-917 (1959)). Subsequently, the phospholipids were loaded on normal phase HPLC column (Ultrasphere silica, 4.6 x 250 mm and 4.6 x 45 mm, Beckman) to fraction the eluted phosphatidylcholine (PC) and lysophosphatidylcholine (lyso-PC), and the amount of phosphorus was determined as previously reported (Saiga, et al., Biochim. Biophys. Acta 1530, 67-76 (2001)).

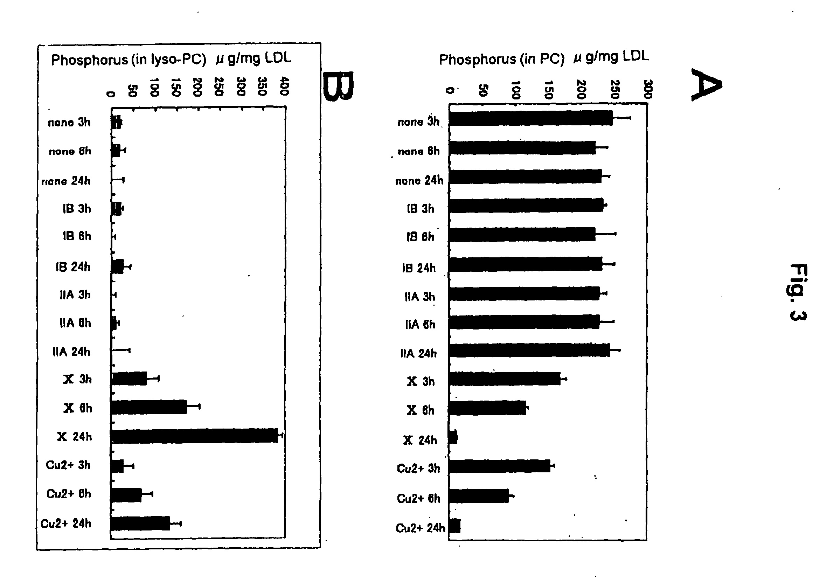

The result showed that treatment with CuSO₄ and group X sPLA₂ decreased PC contents in LDL with time course as shown at the time points of 3, 6, and 24 hours (Fig. 3A), and lyso-PC contents were increased according to the PC decrease (Fig. 3B). Treatment with CuSO₄ caused decreased PC contents in HDL, and increased lyso-PC contents with time course similarly to the case of LDL. Treatment with group X sPLA₂ decomposed PC in HDL much faster than the case of LDL, and 3-hours treatment decreased the content up to 10% of original level, whereas lyso-PC contents were increased according to the PC decrease, and were produced in an amount of 6 times amount of CuSO₄ -treated HDL after 3 hours. On the other hand, treatment with group IB and IIA sPLA₂ showed no significant change in the phospholipid composition (Fig. 3A, B).

### Example 3 Effect of group X sPLA₂ on negative charge of isolated human lipoproteins

LDL and HDL were isolated from human plasma by ultracentrifugation (Havel, et al., J. Clin. Invest. 34, 1345-1353 (1955)). Human group IB, IIA and X sPLA₂ s (50 nmol/L) and CuSO₄ (20 µmol/L) were reacted with LDL and HDL (1mg/ml) at 37°C for 3, 6, and 24 hours in a solution containing 12.5 mmol/L Tris-HCL buffer (pH 8.0), 125 mg/L bovine serum albumin, and 1 mmol/L calcium chloride. Then, a 1 µg sample of LDL and a 2 µg sample of HDL were electrophoresed on agarose gel (Helena Laboratories, TITAN GEL Lipoprotein; 90V, 25 minutes). After the electrophoresis, the gel was dried at 50 to 60°C for about 1 hour, stained with 6 ml of a stain solution (Helena Laboratories, Fat Red 7B agarose solution) for about 3 minutes, and then decolorized with 25 ml of a decolorizing solution (70% methanol). In the experiment for determining the activity of a sPLA₂ inhibitor (indoxam), a COX inhibitor (indomethacin) or a 5-LOX inhibitor (AA-861), those agents were added to the reaction giving a final concentration of 10 µmol/L, virtually simultaneously with the addition of sPLA₂.

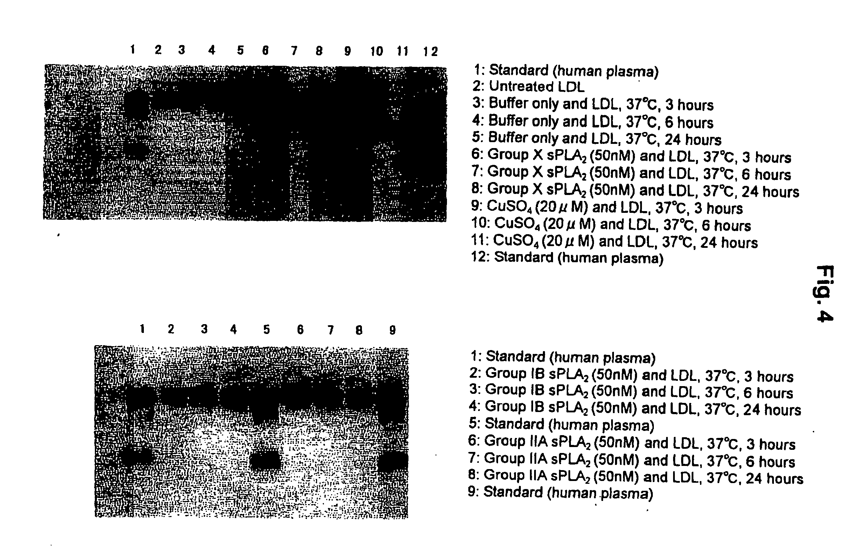

The result is shown in Fig. 4. Treatment of both LDL and HDL with CuSO₄ resulted in a large migrating shift toward the anode with time course compared to the untreated lipo (no data for HDL). Among treatments with human sPLA₂ s, only treatment with group X sPLA₂ resulted in a time-dependent migrating shift toward the anode similar to the treatment with CuSO₄, showing that the change in phospholipid composition caused by the group X sPLA₂ -induced fatty acid release caused the increase in negative charge of each lipoprotein. This type of lipoprotein degeneration induced by group X sPLA₂ was inhibited by a sPLA₂ inhibitor (indoxam), and it was affected neither by a COX inhibitor (indomethacin) nor a 5-LOX inhibitor (AA-861).

### Example 4 Degeneration activity of group X sPLA₂ on apoproteins

LDL and HDL were isolated from human plasma by ultracentrifugation (Havel, et al., J. Clin. Invest. 34, 1345-1353 (1955)). Human group IB, IIA and X sPLA₂ s (50 nmol/L) and CuSO₄ (20 µmol/L) were reacted with LDL and HDL (1mg/ml) at 37°C for 3, 6, and 24 hours in a solution containing 12.5 mmol/L Tris-HCL buffer (pH 8.0), 125 mg/L bovine serum albumin, and 1 mmol/L calcium chloride. After delipidated with a solution of acetone : ethanol (1:1), the reaction mixtures were dissolved in a buffer for electrophoresis (10 mmol/L Tris-HCL buffer (pH 6.8), 2% SDS, 30% Glycerol, 0.1% 2ME, 0.1% BPB), and analyzed by SDS-polyacrylamide gel electrophoresis (SDS-PAGE). In the electrophoresis, each 5 µg of LDL (apoB-100) and of HDL (apoA-1) was loaded on 4% gel and 4-20% gradient gel, respectively.

The result showed that the band of the apoA-1 treated with CuSO₄ disappeared at the original position to be migrated in view of the protein molecular weight, and shifted toward the high molecular weight side as a broad band resemble an aggregation form, whereas the band of the apoB-100 in LDL also shifted toward the high molecular weight side. On the other hand, a part of apoB-100 and apoA-1 treated with group X sPLA₂ shifted toward the high molecular weight side as shown above 24 hours after the treatment, although not as significant as the treatment with CuSO₄. Treatment with group IB and IIA sPLA₂ caused no change compared to the untreated.

### Example 5 Effect of group X sPLA₂ on peroxidation of lipid in lipoproteins

### A. Effect on the production of thiobarbituric acid reactive substances

LDL and HDL were isolated from human plasma by ultracentrifugation (Havel, et al., J. Clin. Invest. 34, 1345-1353 (1955)). Human group IB, IIA and X sPLA₂ s (50 nmol/L) and CuSO₄ (20 µmol/L) were reacted with LDL and HDL (1mg/ml) at 37°C for 3, 6, and 24 hours in a solution containing 12.5 mmol/L Tris-HCL buffer (pH 8.0), 125 mg/L bovine serum albumin, and 1 mmol/L calcium chloride. Then, 500 µl of 20% TCA and 500 µl of thiobarbituric acid (3.35 mg/ml) were added to the preparation of each 10 µg of LDL and HDL in 200 µl of a physiological saline, and the mixture was boiled at 95°C for 60 minutes, according to the known method (Nagano, et al., Proc. Natl. Acad. Sci. USA. 88, 6457-6461, (1991)). After cooling, the thiobarbituric acid reactive substances (TBARS), which is one of the indicators for peroxidation of lipids, was extracted with n-butanol (2 ml), and the fluorescence in the supernatant was determined (excitation wavelength: 515 nm, fluorescence wavelength: 550 nm). TBARS amount was calculated by reference to a standard curve generated using a tetraethoxypropane as a standard.

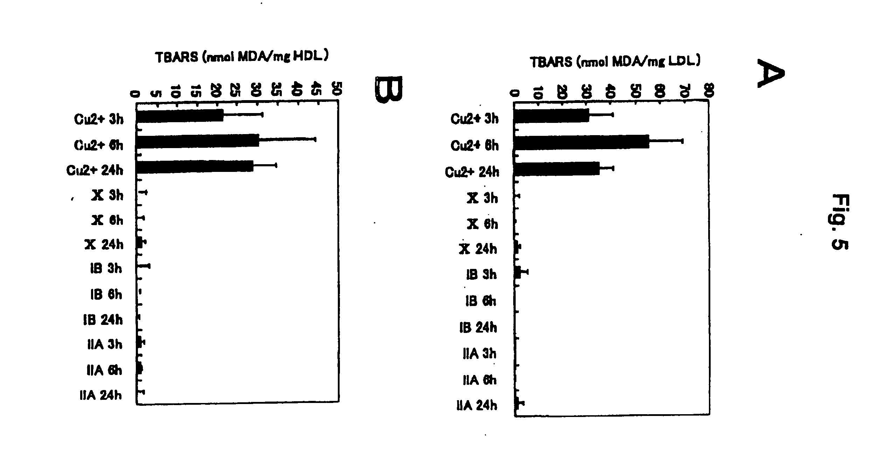

The results are shown in Fig. 5A and B. Significantly elevated amount of TBARS having a peak at 6 hour was observed in both LDL (Fig. 5A) and HDL (Fig. 5B) treated with CuSO₄. On the other hand, no elevated amount of TBARS was observed in the treatment with all sPLA₂ s including group X sPLA₂.

### B. Effect on the production of conjugated diene

LDL and HDL were isolated from human plasma by ultracentrifugation (Havel, et al., J. Clin. Invest. 34, 1345-1353 (1955)). Human group IB, IIA and X sPLA₂ s (50 nmol/L) and CuSO₄ (20 µmol/L) were reacted with LDL and HDL (0.2 mg/ml) at 37°C for 0 to 6 hours in a solution containing 12.5 mmol/L Tris-HCL buffer (pH 8.0), 125 mg/L bovine serum albumin, and 1 mmol/L calcium chloride. Then, the production of conjugated diene, which is one of the indicators for lipid peroxidation, was analyzed by monitoring the changes in absorbance at 234 nm with time course in accordance with the known method (Esterbauer, et al., Free Rad. Res. Comms. 6, 67-75 (1989)).

The result showed that absorbance at 234 nm was increased in the time-dependent manner for 90 minutes from the addition in the treatment of both LDL and HDL with CuSO₄, and then the absorbance was gradually decreased. On the other hand, no change in the absorbance at 234 nm was observed in the treatment with all sPLA₂ s including group X sPLA₂.

The results as described above demonstrated that the degeneration of lipoproteins induced by group X sPLA₂ (fatty acid release, change in phospholipid composition, increased negative charge, polymerization of apoprotein) are similar to the degeneration by oxidation of CuSO₄ treatment, but is not dependent on the oxidation of lipoproteins.

### Example 6 Effect of group X sPLA₂ on uptake of degenerated LDL into mouse peritoneal macrophages

C57BL/6J mice (male, 8 weeks) were injected peritoneally with 2 ml of a 4% thioglycollate solution, and, 4 days later, the celis from the peritoneal fluids were harvested. The cells were plated on a chamber slide, and incubated for 2 hours in the presence of the serum-free medium (BIO WHITTAKER: X-VIVO 15). Then, non-adherent cells were removed by the washing procedure to prepare peritoneal macrophages. The cells were added with 0.2 mg/ml LDL (Sigma) and 50 nmol/L group X PLA₂, and incubated in the serum-free medium for 48 hours. Uptake of LDL into macrophages were confirmed by immobilizing the cells with 4% formaldehyde for 20 minutes, and then staining the lipids with an Oil Red O stain solution. In the experiment for determining the inhibitory activity of a sPLA₂ inhibitor (indoxam), a COX inhibitor (indomethacin) or a 5-LOX inhibitor (AA-861), those agents were added to the reaction giving a final concentration of 10 µmol/L, virtually simultaneously with the addition of sPLA₂.

The results showed that a positive signal for Oil Red O stain was detected in the cells treated with group X sPLA₂, which demonstrated the uptake of LDL into the cells, whereas no positive signal was detected in the untreated cells. This revealed that group X sPLA₂ increased uptake of LDL into macrophages, and has the potential to contribute to the formation of foam cells developed in arteriosclerosis lesions. Effect of group X sPLA₂ on uptake of LDL into the cells was inhibited with a sPLA₂ inhibitor (indoxam), which was added to the cells simultaneously with the addition of group X sPLA₂ and LDL, and it was affected neither by a COX inhibitor (indomethacin) nor a 5-LOX inhibitor (NDGA).

### Example 7 Immunohistochemical analysis of mouse arteriosclerosis lesions using anti- group X sPLA₂ polyclonal antibody

Apolipoprotein E gene-deficient mice, C57BL/6J - Apoe^{t m l U n c} mice (female, 8 weeks) described in The Journal of Clinical Investigation Vol.94, pp.937-945 (1994) were purchased from Jackson Lab., and used as an animal model for arteriosclerosis. As control, C57BL/6 mice (male, 8 weeks) were used. Immediately after the purchase, the animals were grown and fed with the high-fat diet wherein 15.8 % cocoa butter, 1.25 % cholesterol and 0.5 % sodium cholate were supplemented into a conventional pellet, CA-1. Each of mice aged 12 weeks, 17 weeks, and 22 weeks were performed on perfusion expulsion of blood, and the vessels were chemically immobilized with a 4% aqueous paraformaldehyde and dissected to remove the juxtacardiac ascending aorta. The tissue was immersed in the same immobilization liquid overnight, washed with a phosphate buffer, dehydrated conventionally with an aqueous ethanol briefly, and embedded in paraffin wax. Microtome was used to prepare paraffin sections having a thickness of about 5 µm, and then they were mounted on slide glass to make pathological samples for staining. In the immunohistochemistry, each sample slide was immersed in xylol to remove the paraffin wax, reacted with methanol containing 0.3% H₂ O₂ to remove endogenous peroxidases, and then treated with 5% normal goat serum for 20 minutes. Subsequently, the sample slides were immersed in PBS containing 0.1% bovine serum albumin for 30 minutes, and reacted with anti-human group X sPLA₂ rabbit polyclonal antibody (6 µg/mL) at 4°C for 14 hours as described in The Journal of Biological Chemistry Vol.274, No.48, pp.34203-34211 (1999). Then, the slides were washed thoroughly with PBS containing 0.1% polyoxyethylene (20) sorbitan monolaurate (Tween20; Wako Pure Chemical Industries, Ltd.Osaka), reacted with biotinylated goat anti-rabbit IgG antibody for 30 minutes, and treated with a peroxidases-containing avidin-biothin complex reagent (Vector Laboratories), followed by being left for 30 minutes. After washing with PBS, the reaction in a 50 mmol/L Tris-HCl (pH 7.6) buffer containing 200 µg/mL diaminobenzidine and 0.006% H₂ O₂ for 10 minutes developed coloration of the peroxidase activity amplified around the target group X molecules, so that the localization of group X sPLA₂ was visualized in the tissue samples. This analysis will detect the positive signal for group X sPLA₂ molecules as liver-brown pigmentation of diaminobenzidine. In the absorption-neutralization experiment for group X sPLA₂ signal, the antibody was reacted with purified mouse group X sPLA₂ proteins (300 µg/mL) for 2 hours before being added onto the slides, and then the slides were treated with the antibody and the protein reaction. Also, the tissue sample was treated with 0.4% haematoxylin solution to counter-stain the cell nuclei.

The results showed that the positive signal for group X sPLA₂ in the vessels isolated from the apolipoprotein E gene-deficient mice aged 12 weeks, 17 weeks, and 22 weeks was locally detected only at the population of foam cells, which is the initial sign of arteriosclerosis. On the other hand, in the control vessel tissues of normal mice, no initial sign of arteriogenesis was not detected, and no positive signal for group X sPLA₂ was detected. Further, the signal as detected at the arteriosclerosis lesion in the apolipoprotein E gene-deficient mice was disappeared by the absorption-neutralization treatment using mouse group X sPLA₂ protein, suggesting that the positive reaction is specific to group X sPLA₂ molecules. Those positive signals were not detected with IgG prepared from non-immunized rabbits. The results as described above show that group X sPLA₂ molecules are significantly expressed in a high level at the foam cells of vessel lesions of the mouse arteriosclerosis model.

### Example 8 Increase in cholesterol esters within macrophage induced by group X sPLPL₂-degencrated LDL

Group X sPLA₂ (50 nmol/L) was reacted with LDL from human plasma at 37°C for 24 hours in a solution containing 12.5 mmol/L Tris-HCL buffer (pH 8.0), 125 mg/L bovine serum albumin, and 1 mmol/L calcium chloride to prepare LDL degenerated by group X sPLA₂. Peritoneal macrophages were prepared by injecting 2 ml of a 3% thioglycollate solution into C57BL/6J mice (male, 8 weeks) peritoneally, harvesting the cells from the peritoneal fluids 4 days later, plating the cells on a 24-well plate, incubating for 2 hours in the presence of the serum-free medium (BIO WHITTAKER: X-VIVO 15), and then removing non-adherent cells by the washing procedure. The cells were added with 0.2 mg/ml LDL, incubated in the serum-free medium for 48 hours, and allowed to stand in a mixture of hexane and isopropanol (3 : 2) for 30 minutes so as to extract the cholesterol deposited in macrophages. After replacing the solvent with isopropanol, the extracted cholesterols were reacted with 1 unit/ml cholesterol oxidase (Roche), 10 unit/ml peroxidase (Boehringer Mannheim), 40 µg/ml p-hydroxyphenyl acetate (Sigma), and 1 unit/ml cholesterol esterase (TOYOBO) in a 0.1 M phosphate buffer at 37°C for 30 minutes according to the known method (Gamble, et al., J. Lipid. Res. 19, 1068-1070 (1978)), and the total cholesterol (including the free cholesterol and cholesterol esters) was determined by measuring the fluorescence in the solution (excitation wavelength: 305 nm, fluorescence wavelength: 420 nm). Further, the reaction in a reaction mixture free from cholesterol esterase was performed at 37°C for 30 minutes, and only the free cholesterol was determined. Amounts of the total cholesterol and the free cholesterol were calculated by reference to a standard curve generated using cholesterol and cholesterol oleate as standards, and the free cholesterol amount was deduced from the total cholesterol amount to give cholesterol ester amount.

The results showed that the cholesterol ester amount in the uptake of group X sPLA₂-treated LDL was increased significantly compared to that in the uptake of untreated LDL, and thus that the LDL modified by the enzyme has the potential to accelerate the formation of foam cells developed in arteriosclerosis lesions.

### Example 9 Effect of group X sPLA₂-induced degeneration on cholesterol extraction by HDL

Group X sPLA₂ (50 nmol/L) was reacted with HDL from human plasma at 37°C for 3 hours in a solution containing 12.5 mmol/L Tris-HCL buffer (pH 8.0), 125 mg/ L bovine serum albumin, and 1 mmol/L calcium chloride to prepare HDL degenerated by group X sPLA₂. Peritoneal macrophages were prepared by harvesting the washing of peritoneal fluids from ICR mice (female, 12 weeks), plating the same on a 24-well plate, incubating for 2 hours in the presence of the 10% fetal calf serum-containing medium (GIBCO BRL: Dulbecco's Modified Eagle Medium), and then removing non-adherent cells by the washing procedure. The cells were added with 50 µg/ml acetylated LDL (Biomedical Technology Inc.), and incubated for 24 hours to prepare foamy macrophages. After the excess of acetylated LDL in the medium was removed, 100 µg/ml HDL was added to the medium, and then the medium was incubated in the absence of serum for 24 hours. The intracellular cholesterol was extracted and determined, and then the capability of HDL to cause cellular cholesterol efflux from the foam cells was determined.

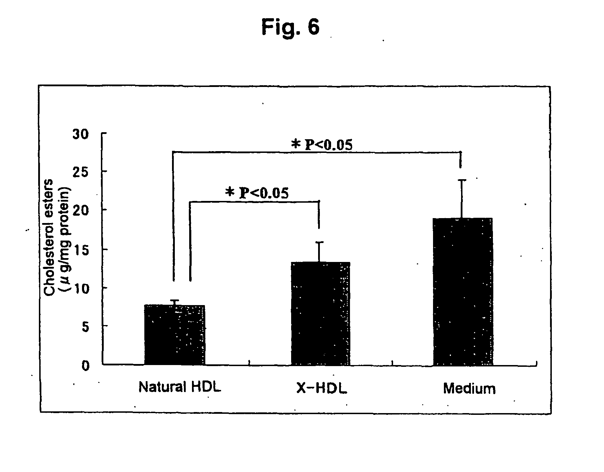

The results showed that the efflux of intracellular cholesterol from the cells treated with group X sPLA₂-degenerated HDL was decreased significantly compared to that from the cells treated with undegenerated HDL. This demonstrated that the degeneration of HDL by the enzyme has the potential to suppress the efflux of cholesterol by HDL in the foam cells developed in arteriosclerosis lesions (Fig. 6).

### Example 10 Effect of group V sPLA₂ on fatty acid release from isolated human lipoproteins

### A. Preparation of cells expressing human group V sPLA₂ and purification of the enzyme from the culture supernatant

cDNA encoding human group V sPLA₂ was obtained by PCR using human heart marathon ready cDNA from CLONTECH as a template. The following primers were used in PCR:
hGV-S: 5'-caaagaacgcgtccaccatgaaaggcctcctcccactggct-3' (SEQ ID NO:1)
hGV-AS: 5'-ctcgctgcggccgcctaggagcagaggatgttgggaaa-3' (SEQ ID NO:2)

hGV-S contains Kozak sequence and a recognition site for restriction enzyme Mlu I. hGV-AS contains a recognition site for restriction enzyme Not I. PCR was conducted at 35 cycles in conditions of 94°C for 0.5 minute, 55°C for 0.5 minute, and 72°C for 2.5 minutes. PCR amplification fragments were digested with Mlu I and Not I, and inserted into the modified pBluescript-SK(-). The base sequence was confirmed using Sequenase Ver.2.0 (USB). Then, group V sPLA₂-His Tag wherein the six His residues were attached to the carboxyl-terminus was constructed by PCR using hGV-S primer and hGV-H6AS primer (5'-ctcgctgcggccgcctaatggtgatggtgatgatgggagcagagga tgttgggaaag-3') (SEQ ID NO: 3), and using human group V sPLA₂ plasmid DNA as a template. The PCR amplification fragments were digested with Sma I and Not I and were replaced with the site corresponding to the group V sPLA₂ plasmid DNA. After confirming the base sequence in the region as newly amplified by PCR, the cDNA was inserted downstream the SR-α promoter of the expression vector for mammal cells. The expression vector was transfected into CHO host cells using a Lipofect AMINE regent (Gibco BRL) according to the instructions of the manufacturer to prepare CHO cells stably expressing human group V sPLA₂. The cells were incubated nearly to confluent phase in α-MEM medium containing 10% fetal calf serum, and the culture supernatant was harvested to give materials for purification.

From the culture supernatant, human group V sPLA₂ was purified using the nickel-chelate HiTrap Chelating HP column (Amersham Pharmacia Biotech) to homogeneous state as migrated as a single bond on SDS-PAGE electrophoresis (molecular weight: about 14 kDa), which then was used in the following analysis for degeneration of lipoproteins.

### B. Effect of group V sPLA₂ on fatty acid release from isolated human lipoproteins

LDL and HDL were isolated from human plasma by ultracentrifugation (Havel, et al., J. Clin. Invest. 34, 1345-1353 (1955)). Human group IIA or V sPLA₂ were reacted with LDL and HDL (1 mg/ml) at 37°C in a solution containing 12.5 mmol/L Tris-HCL buffer (pH 8.0), 125 mg/L bovine serum albumin, and 1 mmol/L calcium chloride, and the fatty acids released were extracted according to the method of Dole (Dole, et al., J. Biol. Chem. 235, 2595-2599 (1960)). The fatty acids were labeled with 9-anthryldiazomethane by the known method (Hanasaki, et al., J. Biol. Chem. 274, 34203-34211 (1999)), and then were determined by detecting fluorescence of the labeled products (fatty acids) eluted from high performance liquid chromatography (HPLC) on reverse-phase column (LichroCART 125-4 Superspher 100 RP-18 column, Merck). In order to examine time-dependent fatty acid release induced by group IIA and V sPLA₂ s, each 50 nmol/L sPLA₂ was used, and the change in the release with time course was traced at the time points of 3, 6, and 24 hour after the sPLA₂ addition. In the experiment for determining the inhibitory activity of a sPLA₂ inhibitor (indoxam), or a COX inhibitor (indomethacin), those agents were added to the reaction giving a final concentration of 10 µmol/L, virtually simultaneously with the addition of sPLA₂.

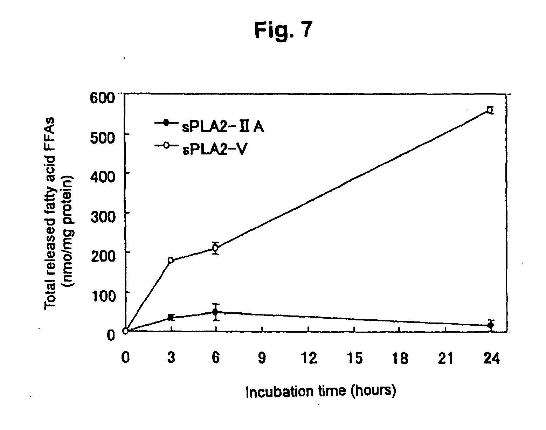

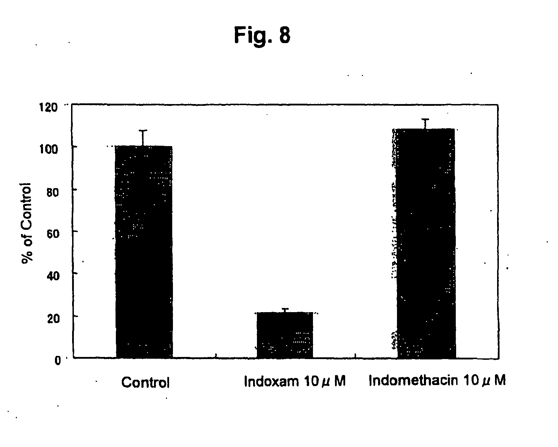

The result showed that group V sPLA₂ released the fatty acids very significantly, and that the fatty acid release from HDL reached a plateau 3 hours after the addition, and continued for 24 hours. On the other hand, the fatty acid release from LDL induced by group V sPLA₂ continued to increase for 24 hours with time course, whereas the fatty acid release induced by group IIA sPLA₂ was shown a quite small amount (Fig. 7). Linoleic acid release of the fatty acid release induced by group V sPLA₂ was inhibited by a sPLA₂ inhibitor (indoxam), and it was not affected by a COX inhibitor (indomethacin) (Fig. 8).

### Example 11 Effect of group V SPLA₂ on the phospholipid composition of lipoproteins

LDL and HDL were isolated from human plasma by ultracentrifugation (Havel, et al., J. Clin. Invest. 34, 1345-1353 (1955)). Human group IIA and V sPLA₂ s (50 nmol/L) were reacted with LDL and HDL (lmg/ml) at 37°C for 3, 6, and 24 hours in a solution containing 12.5 mmol/L Tris-HCL buffer (pH 8.0), 125 mg/L bovine serum albumin, and 1 mmol/L calcium chloride, and then the change in the phospholipid composition of the LDL and HDL was traced by HPLC. Phospholipids were extracted from a 10 µg sample of LDL and a 15 µg sample of HDL according to the method of Bligh, et al. (Bligh, et al., Can. J. Biochem. Physiol. 37, 911-917 (1959)), and subsequently, the phospholipids were loaded on normal phase HPLC column (Ultrasphere silica, 4.6 x 250 mm and 4.6 x 45 mm, Beckman) so that the eluted phosphatidylcholine (PC) and lysophosphatidylcholine (lyso-PC) were fractionated, and the amount of phosphorus was determined as previously reported (Saiga, et al., Biochim. Biophys. Acta 1530, 67-76 (2001)).

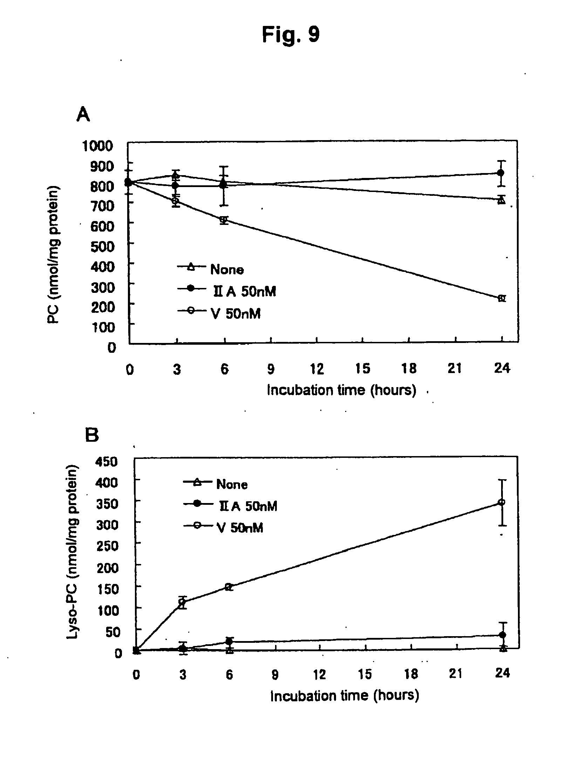

The result showed that treatment of LDL with group V sPLA₂ decreased PC contents with time course as shown at the time points of 3, 6, and 24 hours, and lyso-PC contents were increased according to the PC decrease (Fig. 9A and B). Treatment of HDL with group V sPLA₂ decomposed PC much faster, and 3-hours treatment decreased the content up to about 30% of initial level, whereas lyso-PC contents were also increased according to the PC decrease. On the other hand, treatment with group IIA sPLA₂ showed no significant change in the phospholipid composition.

### Example 12 Degeneration activity of group V sPLA₂ on isolated human lipoproteins

LDL and HDL were isolated from human plasma by ultracentrifugation (Havel, et al., J. Clin. Invest. 34, 1345-1353 (1955)). Human group IIA and V sPLA₂ s (50 nmol/L) were reacted with LDL and HDL (1mg/ml) at 37°C for 3, 6, and 24 hours in a solution containing 12.5 mmol/L Tris-HCL buffer (pH 8:0), 125 mg/L bovine serum albumin, and 1 mmol/ L calcium chloride, and then 1 µg of the LDL and 2 µg of the HDL were spotted on an agarose gel (Helena Laboratories, TITAN GEL Lipoprotein), followed by being electrophoresed at 90V for 25 minutes. After the electrophoresis, the gels were dried at 50 to 60 °C for about one hour, stained with 6 ml of a stain solution (Helena Laboratories, Fat Red 7B agarose solution) for about three minutes, and then decolorized with 25 ml of a decolorizing solution (70% methanol). In the experiment for determining the activity of a sPLA₂ inhibitor (indoxam) or a COX inhibitor (indomethacin), those agents were added to the reaction giving a final concentration of 10 µmol/L, virtually simultaneously with the addition of sPLA₂.

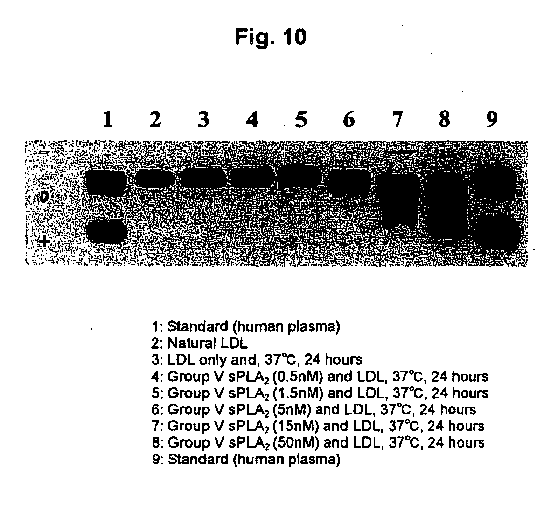

The result showed that treatment of both LDL and HDL with group V sPLA₂ resulted in a large migrating shift toward the anode compared to the untreated. Migration of HDL toward the anode reached a plateau 3 hours after the addition similarly to the fatty acid release, and continued for 24 hours. Migration of LDL toward the anode continued to increase for 24 hours with time course. As shown above, the fatty acid release induced by group V sPLA₂ revealed that negative charge of each lipoprotein was changed (degenerated) (Fig. 10). On the other hand, treatment of each lipoprotein with human group IIA sPLA₂ resulted in no migrating shift toward the anode.

The lipoprotein degeneration induced by group V sPLA₂ was inhibited by a sPLA₂ inhibitor (indoxam), and it was not affected by a COX inhibitor (indomethacin).

### Example 13 Effect of group V sPLA₂ on peroxidation of lipoproteins

### (Effect on the production of thiobarbituric acid reactive substances)

LDL and HDL were isolated from human plasma by ultracentrifugation (Havel, et al., J. Clin. Invest. 34, 1345-1353 (1955)). Human group IIA and V sPLA₂ s (50 nmol/L) were reacted with LDL and HDL (lmg/ml) at 37°C for 24 and 3 hours, respectively, in a solution containing 12.5 mmol/L Tris-HCL buffer (pH 8.0), 125 mg/L bovine serum albumin, and 1 mmol/L calcium chloride, and then 500 µl of 20% TCA and 500 µl of thiobarbituric acid (3.35 mg/ml) were added to the preparation of each 10 µg of LDL and HDL in 200 µl of a physiological saline, followed by boiling at 95°C for 60 minutes in accordance with the method of Nagano et al. (Proc. Natl. Acad. Sci. USA. 88, 6457-6461, (1991)). After cooling, the thiobarbituric acid reactive substances (TBARS), which is one of the indicators for peroxidation of lipids, was extracted with n-butanol (2 ml), and the fluorescence in the supernatant was determined (excitation wavelength: 515 nm, fluorescence wavelength: 550 nm). TBARS amount was calculated by reference to a standard curve generated using a tetraethoxypropane as a standard.

The results showed that no elevated amount of TBARS was observed in all of the treatments with group IIA and V sPLA₂ s.

### Example 14 Effect of group V sPLA₂ on uptake of degenerated LDL into mouse peritoneal macrophages

Peritoneal macrophages were prepared by injecting 2-ml of a 4% thioglycollate solution into C57BL/6J mice (male, 8 weeks) peritoneally, harvesting the peritoneal cells 4 days later, plating the cells on a chamber slide, incubating for 2 hours in the presence of the serum-free medium (BIO WHITTAKER: X-VIVO 15), and then removing non-adherent cells by the washing procedure. The cells were added with 0.2 mg/ml LDL (Sigma), and 50 nmol/L group IIA or V sPLA₂, and the culture was incubated in the serum-free medium for 48 hours. Uptake of LDL into macrophages were confirmed by immobilizing the cells with 4% formaldehyde for 20 minutes, and then staining the lipids with an Oil Red O stain solution. The results showed that a positive signal for Oil Red O stain was detected in the cells treated with group V sPLA₂, which demonstrated the uptake of LDL into the cells, whereas no positive signal was detected in the group IIA sPLA₂ -treated cells and the untreated cells. This revealed that group V sPLA₂ increased uptake of LDL into macrophages, and has the potential to contribute to the formation of foam cells developed in arteriosclerosis lesions.

The following reference examples demonstrate the activity of the preferred compounds of the present invention to inhibit human group V or X sPLA₂.

### REFERENCE EXAMPLE

### A. Preparation of cells expressing human group V or X sPLA₂ and preparation of the culture supernatant thereof

cDNA sequence encoding human group V or X sPLA₂ (Chen et al., J. Biol. Chem, 1994, 269, 2365-2368; and Cupillard et al., J. Biol. Chem, 1997, 272, 15745-15752) was inserted in forward direction downstream the promoter of an expression vector for mammal cells, pSVL SV40 Late Promoter Expression Vector (Amersham Pharmacia Biotech). The expression vector was transfected into CHO host cells using a Lipofect AMINE regent (Gibco BRL) according to the instructions of the manufacturer to provide CHO cells stably expressing human group V or X sPLA₂. The cells were incubated in α-MEM medium containing 10% fetal calf serum for 3 days, and the culture supernatant was used to determine the activity of the enzymes.

### B. Assay for inhibitory activity

The following chromogenic assay is used to identify and evaluate inhibitors of group V or X sPLA₂. The assay has been adapted for high volume screening using 96 well microtiter plates. A general description of this assay is found in the article, "Analysis of Human Synovial Fluid Phospholipase A2 on Short Chain Phosphatidylcholine-Mixed Micelles: Development of a Spectrophotometric Assay Suitable for a Microtiterplate Reader", by Laure. J. Reynolds, Lori L. Hughes and Edward A Dennis, Analytical Biochemistry, 204, pp 190-197,1992.

A test compound (or a solvent blank) was added according to the predetermined arrangement of the plate, and diheptanoyl thio PC (1mM) was reacted with human group V or X sPLA₂ in the presence of Triton X-100 (0.3mM), 5,5'-dithiobis-(2-nitrobenzoic acid) (125 µM) in a Tris buffer (25mM, pH7.3), CaCl₂ (10mM), KCl (100mM), bovine serum albumin. Absorbance at 405 nm is read to estimate the inhibitory activity.

The reaction with human group V sPLA₂ was performed in 40 µl/well at 40°C for 45 minutes, and the reaction with human group X sPLA₂ was performed in 15 µl/well at 40°C for 30 minutes.

IC₅₀ values were determined by plotting log concentrations of the test compounds described in Tables 1-4 versus inhibition values in the range from 10-90% inhibition.

The results of group V sPLA₂ inhibition tests are displayed in Table 5, and the results of group X sPLA₂ inhibition tests are displayed in Table 6:

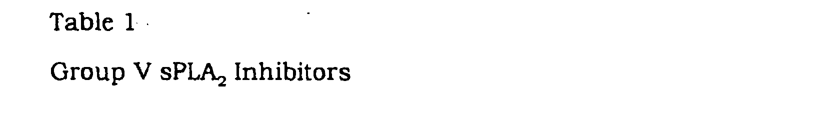

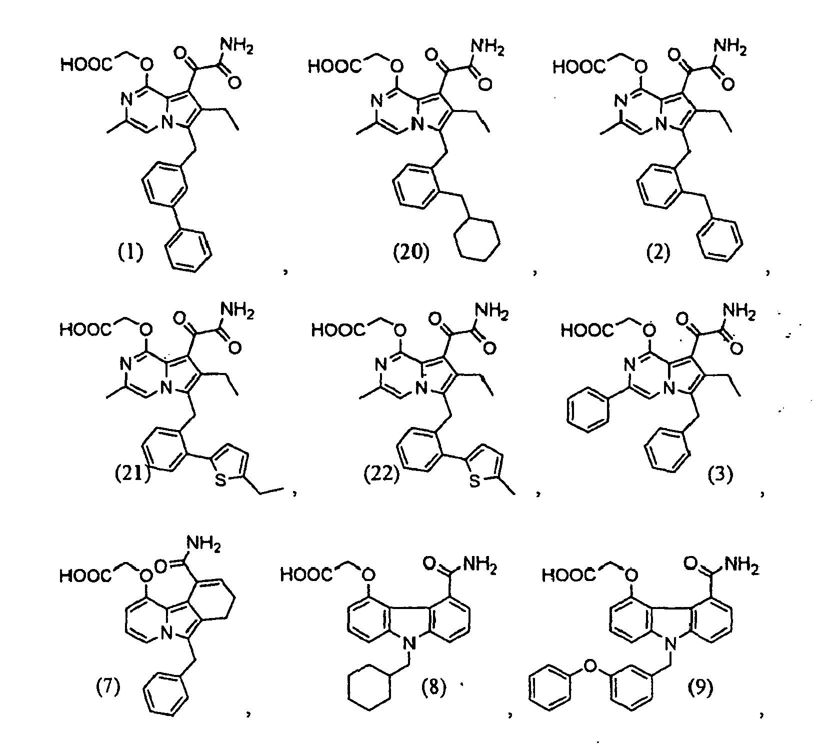

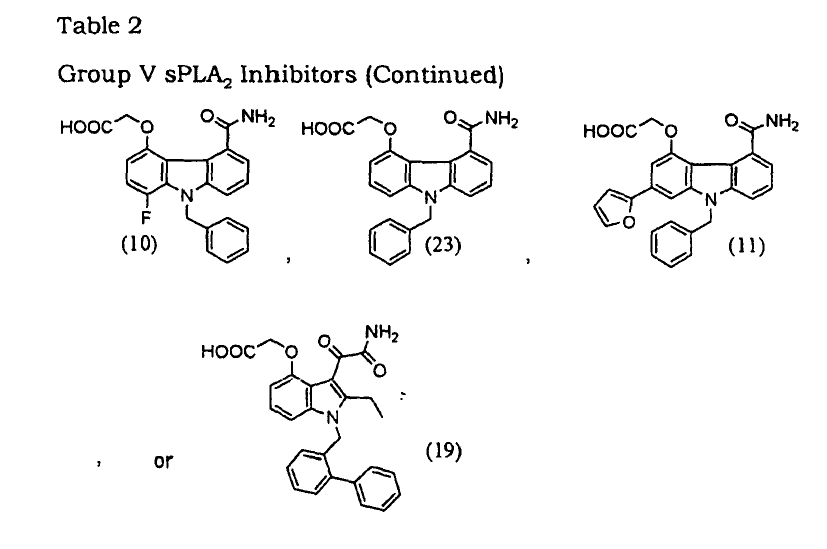

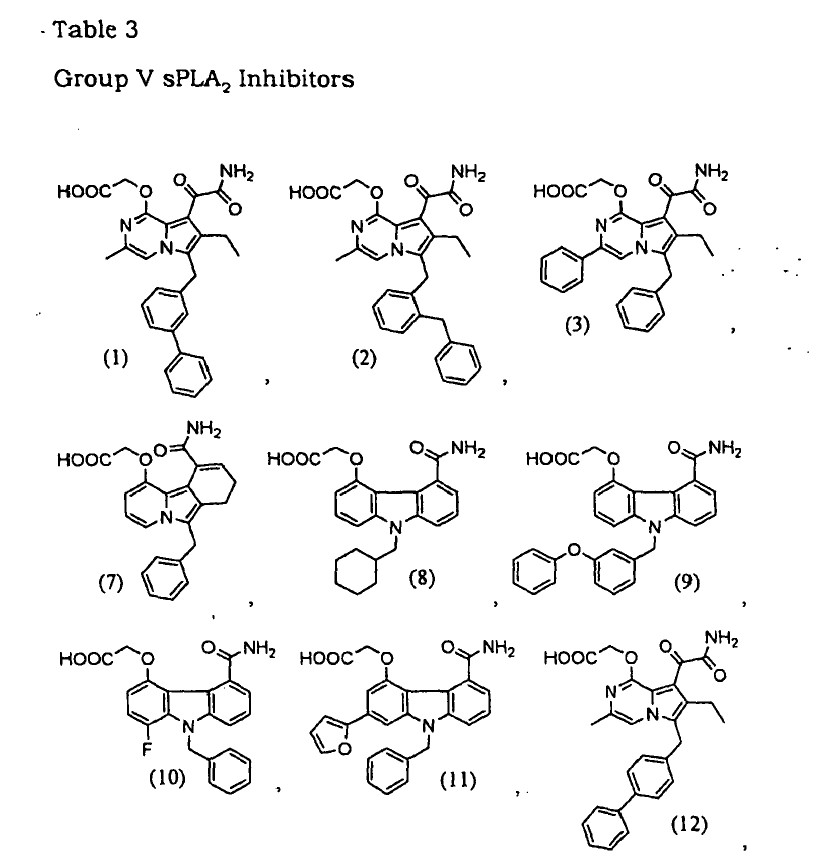

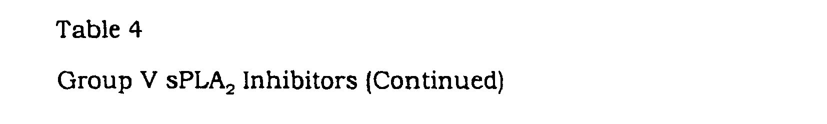

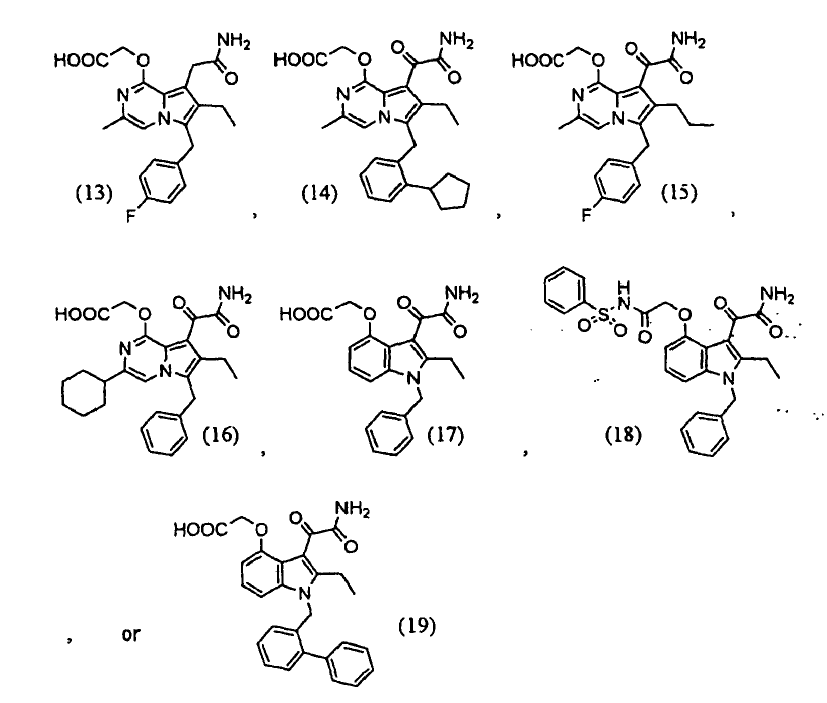

**Table 5**

| | | | | |
|---|---|---|---|---|
| The results of group V sPLA₂ Inhibition Tests | | | | |

| Compound No. | IC₅₀(nM) | Compound No. | IC₅₀(nM) | |
|---|---|---|---|---|
| 1 | 12 | 7 | 2 | |
| 20 | 11 | 8 | 3 | |
| 2 | 13 | 9 | 1 | |
| 21 | 5 | 10 | 7 | |
| 22 | 13 | 23 | 8 | |
| 3 | 5 | 11 | 2 | |
| | | 19 | 6 | |

**Table 6**

| | | | | |
|---|---|---|---|---|
| The results of group X sPLA₂ Inhibition Tests | | | | |

| Compound No. | IC₅₀(nM) | Compound No. | IC₅₀(nM) | |
|---|---|---|---|---|
| 1 | 10 | 12 | 16 | |
| 2 | 10 | 13 | 19 | |
| 3 | 5 | 14 | 9 | |
| 7 | 5 | 15 | 17 | |
| -8 | 3 | 16 | 7 | |
| 9 | 13 | 17 | 12 | |
| 10 | 12 | 18 | 16 | |
| 11 | 10 | 19 | 26 | |

### FORMULATION EXAMPLES

The following Formulation Examples 1 to 8 are mere illustration, but not intended to limit the scope of the invention. The term "active ingredient" means a compound that inhibits group V and/or X sPLA₂, a prodrug thereof, a pharmaceutical acceptable salt of them, or a solvate thereof.

### Formulation Example 1

A hard gelatin capsule is prepared using the following ingredients:

| | Dose (mg/capsule) |
|---|---|
| Active ingredient | 250 |
| Starch, dried | 200 |
| Magnesium stearate | 10 |
| Total | 460 mg |

### Formulation Example 2

A tablet is prepared using the following ingredients:

| | Dose (mg/tablet) |
|---|---|
| Active ingredient | 250 |
| Cellulose, microcrystals | 400 |
| Silicon dioxide, fumed | 10 |
| Stearic acid | 5 |
| Total | 665 mg |

The ingredients are blended and compressed to form tablets each weighing 665 mg.

### Formulation Example 3

An aerosol solution is prepared containing the following ingredients:

| | Weight |
|---|---|
| Active ingredient | 0.25 |
| Ethanol | 25.75 |
| Propellant 22 (chlorodifluoromethane) | 74.00 |
| Total | 100.00 |

The active ingredient is mixed with ethanol and the mixture added to a portion of the propellant 22 was cooled to -30°C and transferred to a filling device. The required amount is then fed to a stainless steel container and diluted with the reminder of the propellant. The valve units are then fitted to the container.

### Formulation Example 4

Tablets, each containing 60 mg of an active ingredient, are made as follows.

| | |
|---|---|
| Active ingredient | 60 mg |
| Starch | 45 mg |
| Microcrystals cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch, and cellulose, all of which are passed through a No. 45 mesh U.S. sieve are mixed thoroughly. The aqueous solution containing polyvinylpyrrolidone is mixed with the resultant powder, and the mixture then is passed through a No. 14 mesh U.S. sieve. The granules so produced are dried at 50°C and passed through a No. 18 mesh U.S. sieve. The sodium carboxymethyl starch, magnesium stearate, and talc, previously passed through No. 60 mesh U.S. sieve, are then added to the granules which, after mixing, are compressed on a tablet machine to yield tablets each weighing 150 mg.

### Formulation Example 5

Capsules, each containing 80 mg of active ingredient, are made as follows:

| | |
|---|---|
| Active ingredient | 80 mg |
| Starch | 59 mg |
| Microcrystals cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

### Formulation Example 6

Suppositories, each containing 225 mg of active ingredient, are made as follows:

| | |
|---|---|
| Active ingredient | 225 mg |
| Saturated fatty acid glycerides | 2000 mg |
| Total | 2225 mg |

The active ingredient is passed through a No. 60 mesh U.S. sieve and suspended in the saturated fatty acid glycerides previously melted using the minimum heat necessary. The mixture is then poured into a suppository mold of nominal 2g capacity and allowed to cool.

### Formulation Example 7

Suspensions, each containing 50 mg of active ingredient, are made as follows:

| | |
|---|---|
| Active ingredient | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The active ingredient is passed through a No. 45 U.S. sieve, and mixed with the sodium carboxymethyl cellulose and syrup to form a smooth paste. The benzoic acid solution, flavor and color are diluted with a portion of the water and added, with stirring. Sufficient water is then added to produce the required volume.

### Formulation Example 8

An intravenous formulation may be prepared as follows:

| | |
|---|---|
| Active ingredient | 100 mg |
| Isotonic saline | 1000 ml |

The solution of the above ingredients is generally administered intravenously to a subject at a rate of 1 ml per minute.

### INDUSTRIAL APPLICABILITY

As described above, the inventors of the present invention found for the first time that group V and X sPLA₂s are responsible for the onset and development of arteriosclerosis by demonstrating that these enzymes degenerate serum lipoproteins, and that these enzymes are expressed at arteriosclerosis lesions. The invention is based on the findings. The inventors examined the inhibitory activity of sPLA₂ inhibitors on lipoprotein degeneration induced by group V and X sPLA₂s, and showed that such compounds are useful in the treatment of ischemic disease based on arteriosclerosis. Specifically, the invention is applicable to medicaments for treatment and prevention of ischemic disease based on arteriosclerosis due to the inhibition of lipoprotein degeneration induced by group V and/or X spLA₂s.

The present invention can also be described as set out in the following numbered clauses.
1. A pharmaceutical composition for suppressing degeneration of serum lipoproteins, which comprises a group V or X sPLA₂-inhibiting compound as an active ingredient.
2. A pharmaceutical composition for treating or preventing arteriosclerosis, which comprises a group V and/ or X sPLA₂-inhibiting compound as an active ingredient.
3. A pharmaceutical composition for treating or preventing an ischemic disease based on arteriosclerosis, which comprises a group V and/or X sPLA₂-inhibiting compound as an active ingredient.
4. The pharmaceutical composition of any one of clauses 1-3, in which the group V and/or X sPLA₂-inhibiting compound is shown by general formula (I): 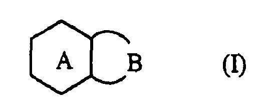 in which
   ring A is: 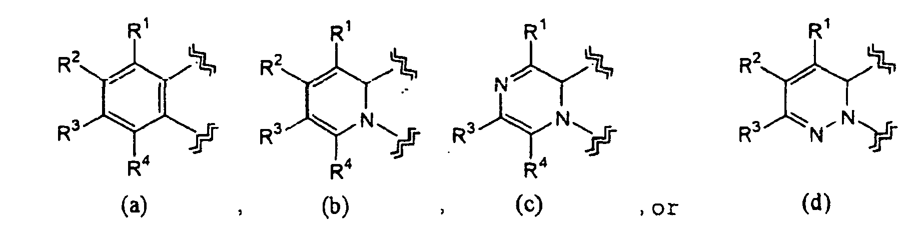 in which either one of R¹ and R² is a group of formula: -(L¹)-(acidic group) wherein L¹ is a linker group to the acidic group, and the length of the linker is 1-5, and the other is a hydrogen atom, a non-interfering substituent, or -(L¹)-(acidic group) wherein L¹ is as defined above; and
   each R³ and R⁴ is independently a hydrogen atom, a non-interfering substituent, a carbocyclic group, a carbocyclic group substituted by a non-interfering substituent, a heterocyclic group, or a heterocyclic group substituted by a non-interfering substituent; and
   -B- is a group of (e)-(h): 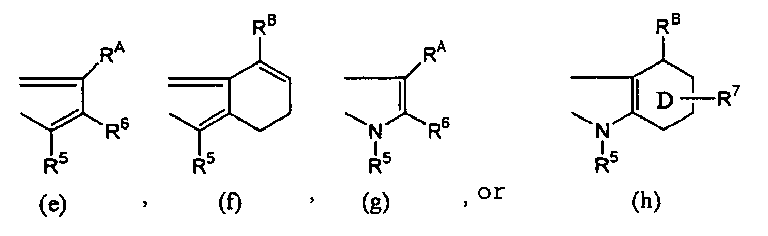 in which R⁵ is a substituent selected from a group consisting of (j) a group of a C1-C20 alkyl, a C2-C20 alkenyl, a C2-C20 alkynyl, a carbocyclic group, or a heterocyclic group; (k) a group of (j) as described above that is substituted by one or more non-interfering substituents each of which is independently selected; or a group of formula: -(L²)-R⁸
   in which L² is a divalent linker group of 1-18 atoms selected from a hydrogen atom, a nitrogen atom, a carbon atom, an oxygen atom and a sulfur atom, and R⁸ is a group selected from (j) and (k);
   R⁶ is a hydrogen atom, a halogen, a C1-C3 alkyl, a C3-C4 cycloalkyl, a C3-C4 cycloalkenyl, a C1-C3 alkyloxy, or a C1-C3 alkylthio;
   R⁷ is a hydrogen atom or a non-interfering substituent;
   R^{A} is a group of formula: 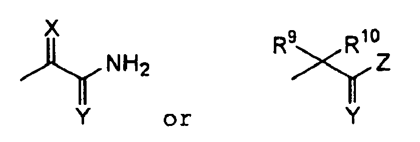 in which each R⁹ and R¹⁰ is independently a hydrogen atom, a C 1-C3 alkyl, or a halogen; each X and Y is independently an oxygen atom or a sulfur atom; and Z is -NH₂ or -NHNH₂;
   R^{B} is -CONH₂ or -CONHNH₂; and
   ring D is a cyclohexene ring or a benzene ring; provided that, when -B- is a group of (e) or (f), then ring A is a ring of (b), (c), or (d);
   or a prodrug thereof or a pharmaceutically acceptable salt of them or a solvate of them.
5. The pharmaceutical composition in clause 4, in which the group V and/or X sPLA₂-inhibiting compound is shown by general formula (I) in which
   R¹ is a hydrogen atom or a group of formula: -(L³)-R¹¹ wherein L³ is -OCH₂-, -SCH₂-, -NH-CH₂-, -CH₂-CH₂-, -O-CH(CH₃)-, or -O-CH-(CH₂CH₂C₆C₅)-; and R¹¹ is -COOH, -CONHSO₂C₆H₅, -SO₃H, or -P(O)(OH)₂; and
   R² is a hydrogen atom or a group of formula: -(L⁴)-R¹² wherein L⁴ is a group of formula: 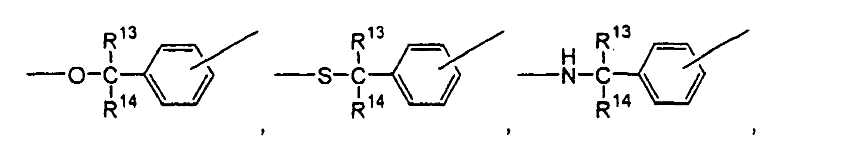 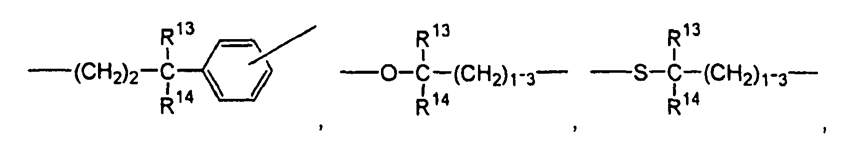 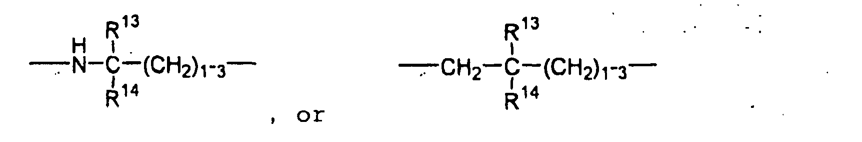 in which each R¹³ and R¹⁴ is independently a hydrogen atom, a C1-C10 alkyl, a C1-C10 aralkyl, a carboxy, an alkyloxycarbonyl, or a halogen; and R¹² is -COOH, -SO₃H, or -P(O)(OH)₂;
   provided that R¹ and R² are not a hydrogen atom, simultaneously;
   or a prodrug thereof or a pharmaceutically acceptable salt of them or a solvate of them.
6. The pharmaceutical composition in clause 4 or 5, in which the group V and/or X sPLA₂-inhibiting compound is shown by general formula (I) in which
   R³ is a hydrogen atom, a C1-C6 alkyl, a C3-C6 cycloalkyl, an aryl, or a heterocyclic group, and R⁴ is a hydrogen atom or a halogen; or a prodrug thereof or a pharmaceutically acceptable salt of them or a solvate of them.
7. The pharmaceutical composition in any one of clauses 4 to 6, in which the group V and/or X sPLA₂-inhibiting compound is shown by general formula (I) in which
   R⁵ is a group of -(CH₂)₁₋₆-R¹⁵ wherein R¹⁵ is a group of formula: 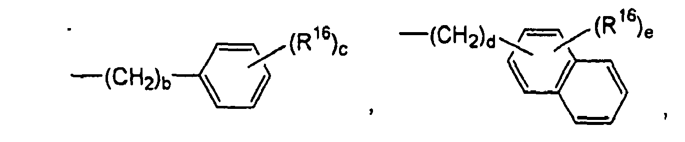 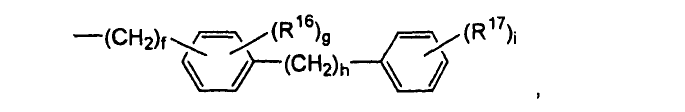 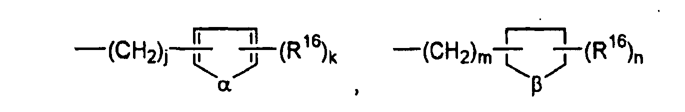 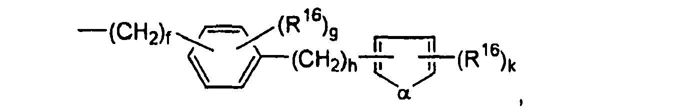 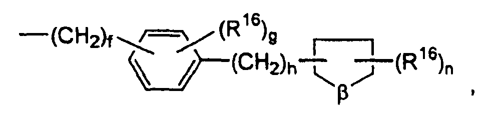 or 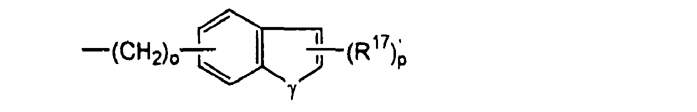 in which each b, d, f, h, j, m, and o is independently an integer of 0 to 2; each R¹⁶ and R¹⁷ is a group selected independently from a halogen, a C1-C10 alkyl, a C1-C10 alkyloxy, a C1-C10 alkylthio, an aryloxy, a phenyl, and a C1-C10 haloalkyl; α is an oxygen atom or a sulfur atom; β is -CH₂- or -(CH₂)₂-; γ is an oxygen atom or a sulfur atom; c, i, and p are an integer of 0 to 5; e is an integer of 0 to 7; g is an integer of 0 to 4; and each k and n is independently an integer of 0 to 3;
   or a prodrug thereof or a pharmaceutically acceptable salt of them or a solvate of them.
8. The pharmaceutical composition of clause 7, in which the group V and/or X sPLA₂-inhibiting compound is shown by general formula (I) in which
   R⁵ is a group of -CH₂-R¹⁸ wherein R¹⁸ is a group of formula: 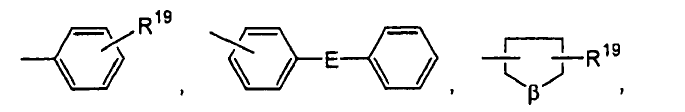 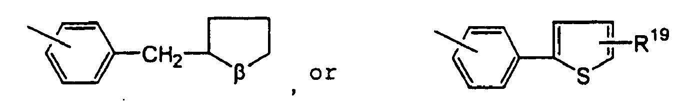 in which β is -CH₂- or -(CH₂)₂-; R¹⁹ is a hydrogen atom, a C1-C3 alkyl, or a halogen; and E is a single bond, -CH₂-, or -O-;
   or a prodrug thereof or a pharmaceutically acceptable salt of them or a solvate of them.
9. The pharmaceutical composition in any one of clauses 4 to 8, in which the group V and/or X sPLA₂-inhibiting compound is shown by general formula (I) in which R¹ is -OCH₂COOH; or a prodrug thereof or a pharmaceutically acceptable salt of them or a solvate of them.
10. The pharmaceutical composition of any one of clauses 4 to 9, in which the group V and/or X sPLA₂-inhibiting compound is shown by general formula (I) in which R² is a hydrogen atom; or a prodrug thereof or a pharmaceutically acceptable salt of them or a solvate of them.
11. The pharmaceutical composition of any one of clauses 4 to 10, in which the group V and/or X sPLA₂-inhibiting compound is shown by general formula (I) in which R⁶ is a C1-C3 alkyl; or a prodrug thereof or a pharmaceutically acceptable salt of them or a solvate of them.
12. The pharmaceutical composition of any one of clauses 4 to 11, in which the group V and/or X sPLA₂-inhibiting compound is shown by general formula (I) in which R^{A} is -C₂CONH₂ or -COCONH₂; or a prodrug thereof or a pharmaceutically acceptable salt of them or a solvate of them.
13. The pharmaceutical composition of any one of clauses 1 to 3, in which the group V and/or X sPLA₂-inhibiting compound is shown by the formula: 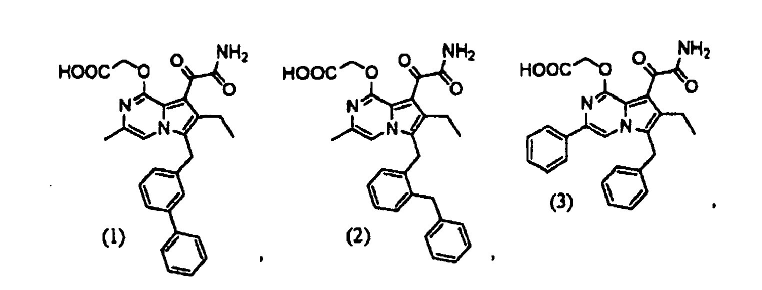 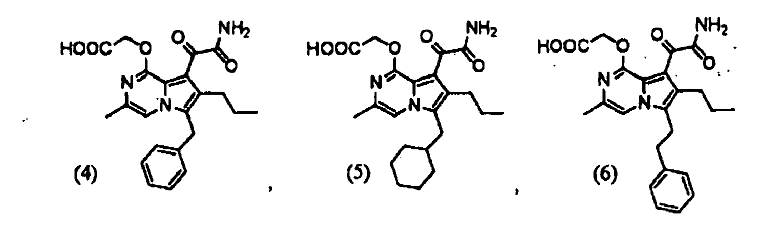 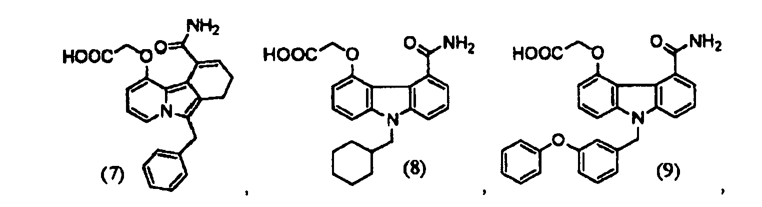 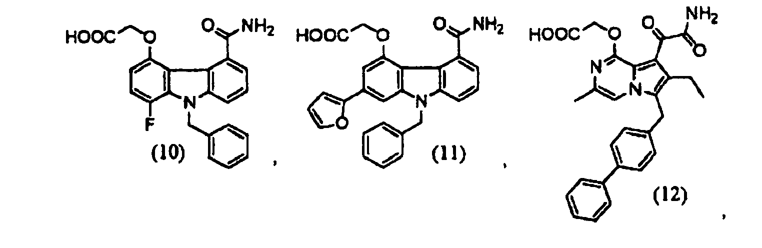 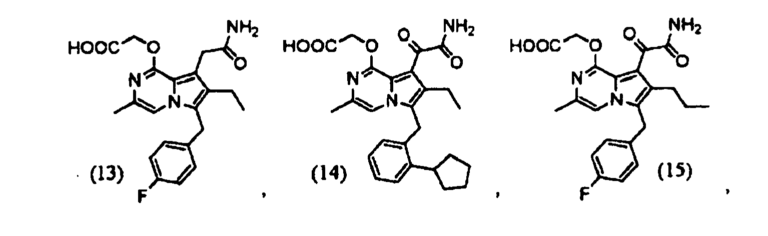 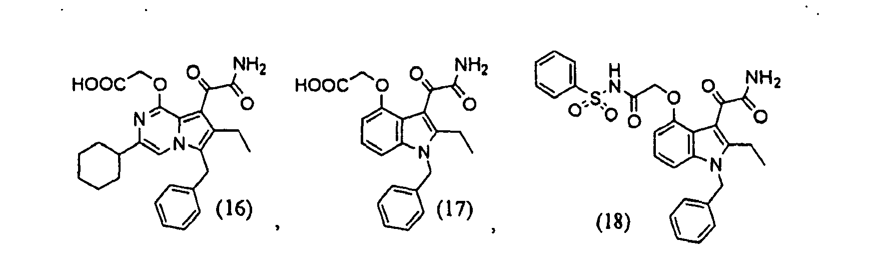 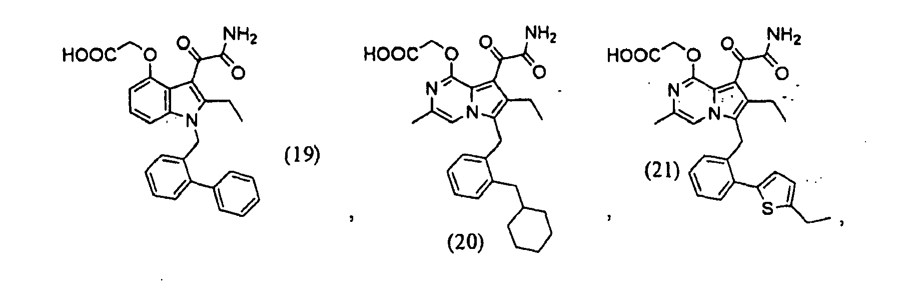 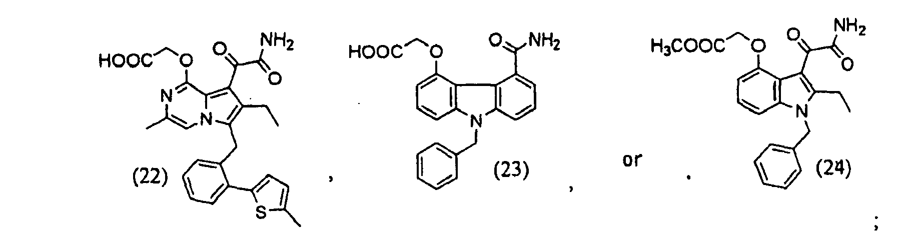 or a prodrug thereof or a pharmaceutically acceptable salt of them or a solvate of them.
14. The pharmaceutical composition of any one of clauses 1 to 3, in which the group V sPLA₂-inhibiting compound is shown by the formula: 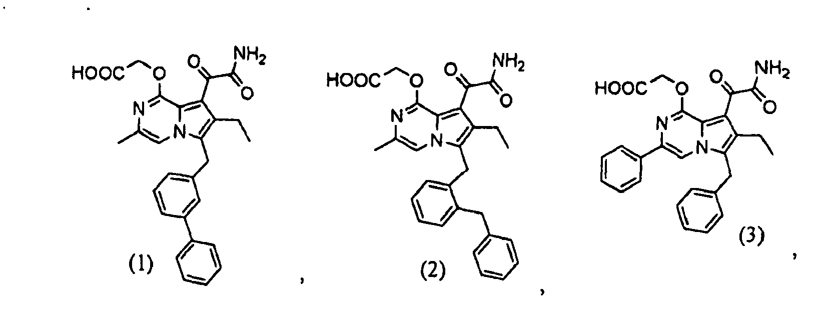 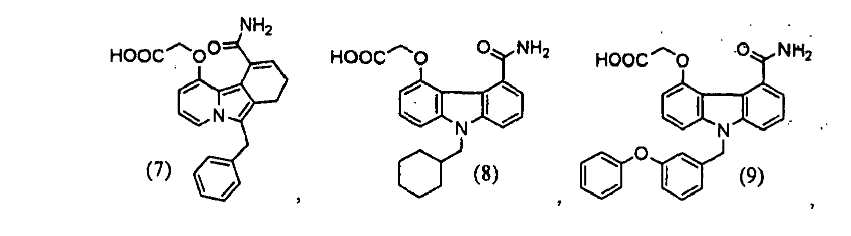 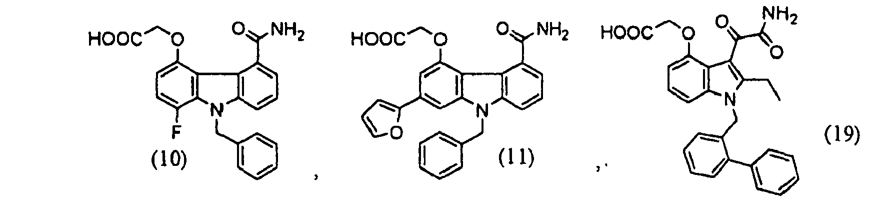 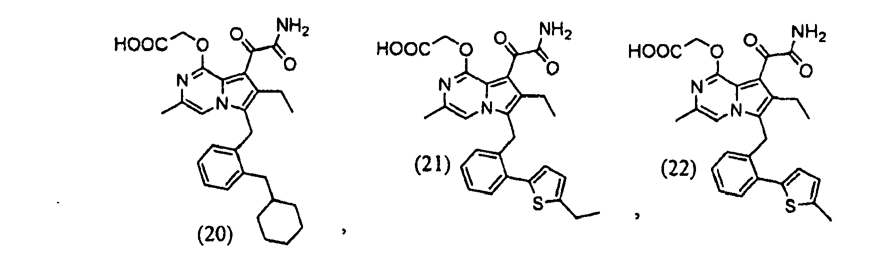 or 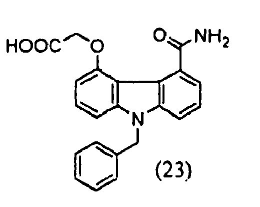 or a prodrug thereof or a pharmaceutically acceptable salt of them or a solvate of them.
15. The pharmaceutical composition of any one of clauses 1 to 3, in which the group X sPLA₂-inhibiting compound is shown by the formula: 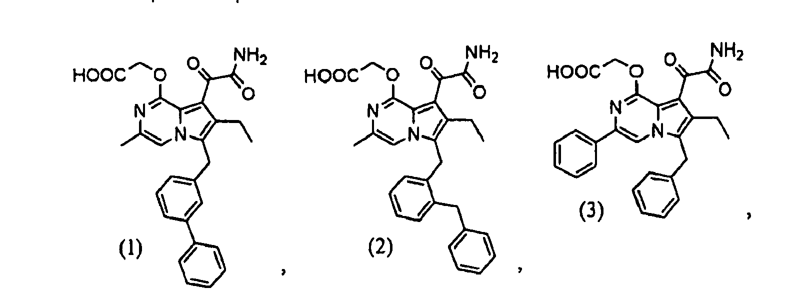 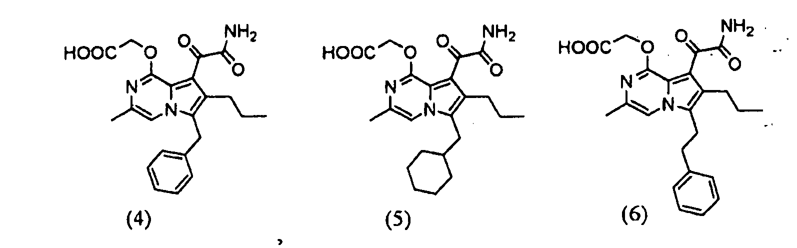 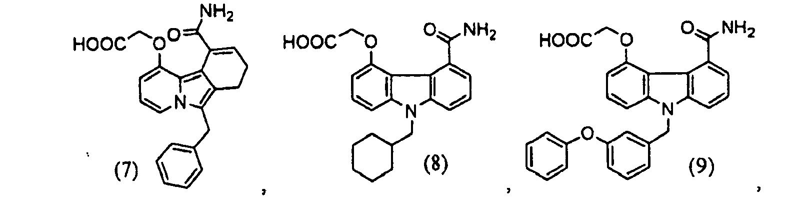 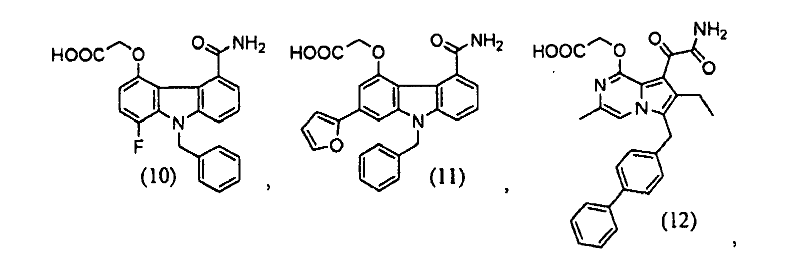 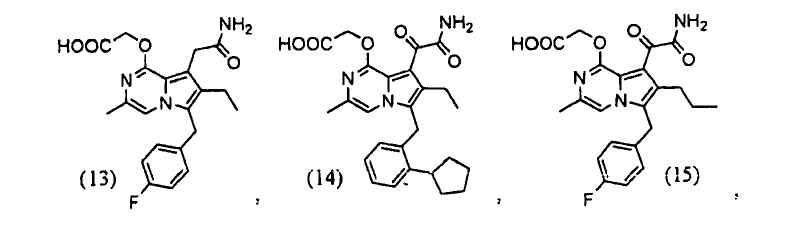 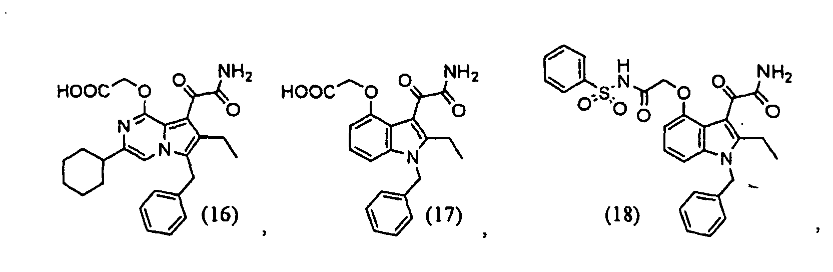 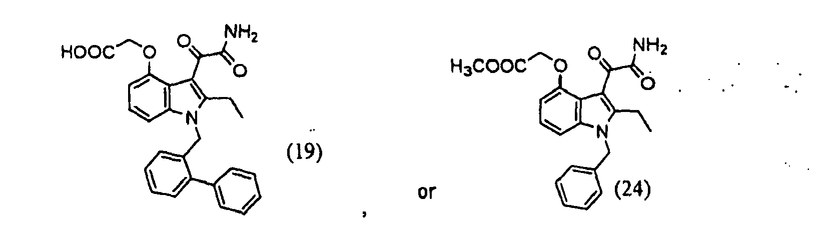 or a prodrug thereof or a pharmaceutically acceptable salt of them or a solvate of them.
16. Use of a group V and/or X sPLA₂-inhibiting compound for preparation of a medicament for treating or preventing arteriosclerosis or an ischemic disease based on arteriosclerosis.
17. The use in clause 16 in which the group V and/or X sPLA₂-inhibiting compound is a compound of any one of clauses 4 to 15.
18. A method for treating or preventing arteriosclerosis or an ischemic disease based on arteriosclerosis in a mammal including a human, which comprises administrating to said mammal a therapeutically effective amount of a group V and/or X sPLA₂-inhibiting compound, thereby alleviating their symptoms.
19. The method in clause 18, in which the group V and/or X sPLA₂-inhibiting compound is a compound of any one of clauses 4 to 15.

## Claims

1. A pharmaceutical composition comprising a group V or X sPLA₂-inhibiting compound represented by general formula (I): 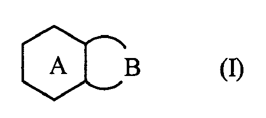 wherein
ring A is: 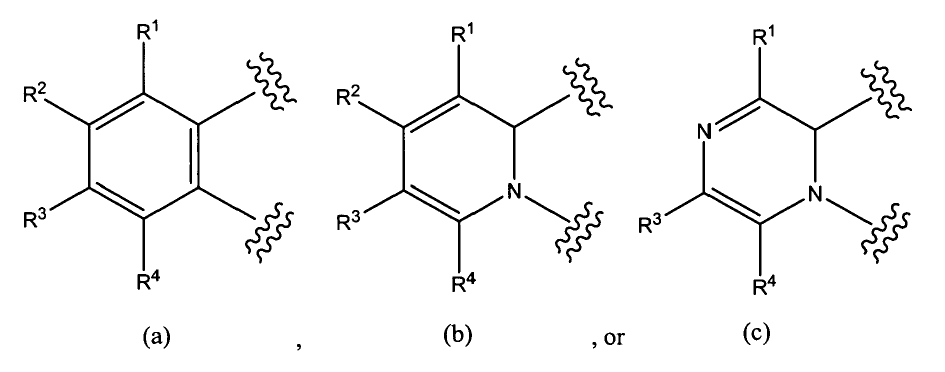 in which either one of R¹ and R² is a group of formula: - (L¹)-(acidic group) wherein L¹ is a linker group to the acidic group, and the length of the linker is 1-5, and the other is a hydrogen atom, a non-interfering substituent, or -(^{L}1)-(acidic group) wherein L¹ is as defined above; and
each R³ and R⁴ is independently a hydrogen atom, a non-interfering substituent, a carbocyclic group, a carbocyclic group substituted by a non-interfering substituent, a heterocyclic group, or a heterocyclic group substituted by a non-interfering substituent; and
-B- is a group of (e)-(h): 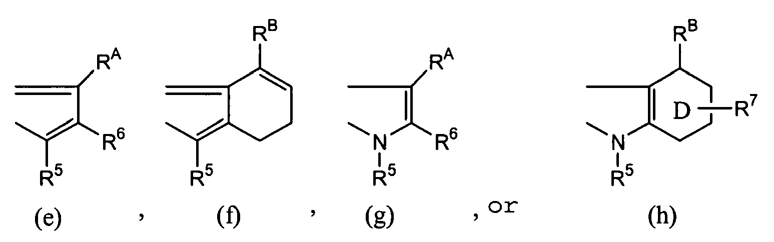 in which R⁵ is a substituent selected from a group consisting of (j) a group of a C1-C20 alkyl, a C2-C20 alkenyl, a C2-C20 alkynyl, a carbocyclic group, or a heterocyclic group; (k) a group of (j) as described above that is substituted by one or more non-interfering substituents each of which is independently selected; or a group of formula: -(L²)-R⁸ in which L² is a divalent linker group of 1-18 atoms selected from a hydrogen atom, a nitrogen atom, a carbon atom, an oxygen atom and a sulfur atom, and R⁸ is a group selected from (j) and (k);
R⁶ is a hydrogen atom, a halogen, a C1-C3 alkyl, a C3-C4 cycloalkyl, a C3-C4 cycloalkenyl, a C1-C3 alkyloxy, or a C1-C3 alkylthio;
R⁷ is a hydrogen atom or a non-interfering substituent;
R^{A} is a group of formula: 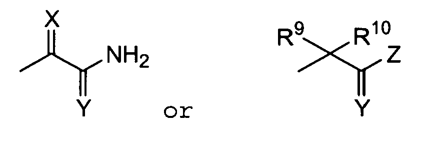 in which each R⁹ and R¹⁰ is independently a hydrogen atom, a C1-C3 alkyl, or a halogen; each X and Y is independently an oxygen atom or a sulfur atom; and Z is -NH₂ or -NHNH₂;
R^{B} is -CONH₂ or -CONHNH₂; and
ring D is a cyclohexene ring or a benzene ring; provided that, when -B- is a group of (e) or (f), then ring A is a ring of (b), or (c) ;
or a pharmaceutically acceptable C1 - C6 alkylester, salt, or solvate thereof; and
one or more pharmaceutically acceptable carriers, diluents, or additives
for use in suppressing degeneration of serum lipoproteins and/or treating or preventing arteriosclerosis and/or treating or preventing an ischemic disease based on arteriosclerosis.

2. The pharmaceutical composition of claim 1 wherein
R¹ is a hydrogen atom or a group of formula: -(L³)-R¹¹ wherein L³ is -OCH₂-, -SCH₂-, -NH-CH₂-, -CH₂-CH₂-, -O-CH(CH₃), or -O-CH-(CH₂CH₂C₆H₅)-; and R¹¹ is -COOH, -CONHSO₂C₆H₅, -SO₃H, or -P(O)(OH)₂; and
R² is a hydrogen atom or a group of formula: -(L⁴)-R¹² wherein L⁴ is a group of formula: 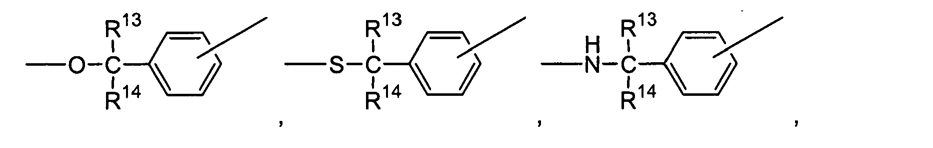 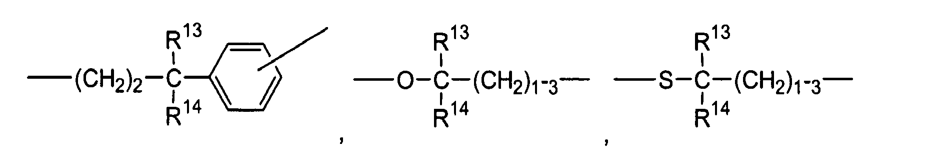 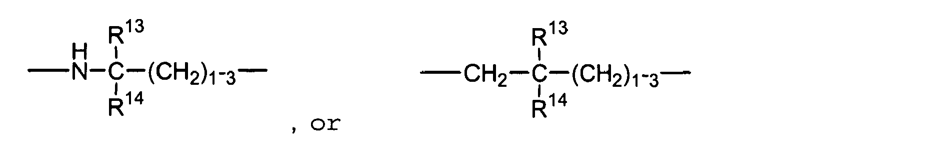 in which each R¹³ and R¹⁴ is independently a hydrogen atom, a C1-C10 alkyl, a C1-C10 aralkyl, a carboxy, an alkyloxycarbonyl, or a halogen; and R¹² is -COOH, -SO₃H, or - P(O)(OH)₂;
provided that R¹ and R² are not a hydrogen atom, simultaneously.

3. The pharmaceutical composition of any one of claims 1 to 2, wherein
R³ is a hydrogen atom, a C1-C6 alkyl, a C3-C6 cycloalkyl, an aryl, or a heterocyclic group, and R⁴ is a hydrogen atom or a halogen.

4. The pharmaceutical composition of any of claims 1 to 3, wherein
R⁵ is a group of -(CH₂)₁₋₆-R¹⁵ wherein R¹⁵ is a group of formula: 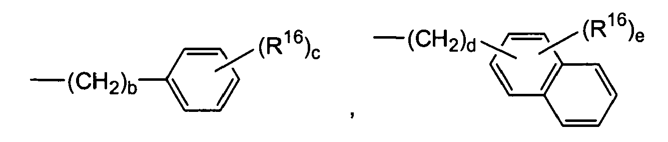 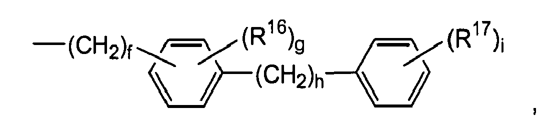 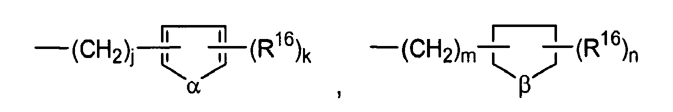 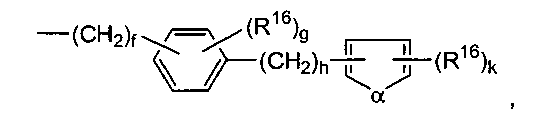 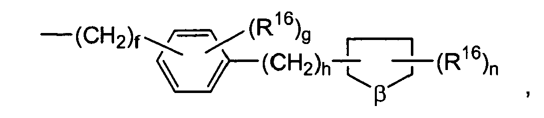 or 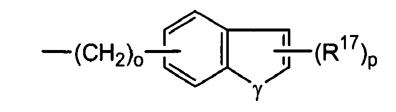 in which each b, d, f, h, j, m, and o is independently an integer of 0 to 2; each R¹⁶ and R¹⁷ is a group selected independently from a halogen, a C1-C10 alkyl, a C1-C10 alkyloxy, a C1-C10 alkylthio, an aryloxy, a phenyl, and a C1-C10 haloalkyl; α is an oxygen atom or a sulfur atom; *β* is -CH₂- or -(CH2)₂-; γ is an oxygen atom or a sulfur atom; c, i, and p are an integer of 0 to 5; e is an integer of 0 to 7; g is an integer of 0 to 4; and each k and n is independently an integer of 0 to 3;

5. The pharmaceutical composition of any one of claims 1 to 4, wherein R⁵ is a group of -CH₂-R¹⁸ wherein R¹⁸ is a group of formula: 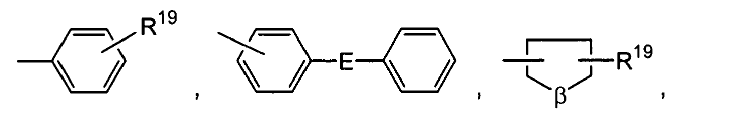 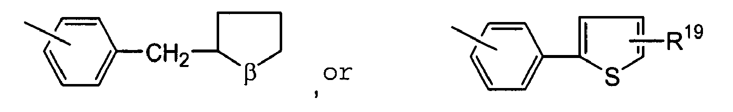 in which *β* is -CH₂- or -(CH₂)₂-; R¹⁹ is a hydrogen atom, a C1-C3 alkyl, or a halogen; and E is a single bond, -CH₂-, or -O-.

6. The pharmaceutical composition of any one of claims 1 to 5, wherein R¹ is -OCH₂COOH.

7. The pharmaceutical composition of any one of claims 1 to 6, wherein R² is a hydrogen atom.

8. The pharmaceutical composition of any one of claims 1 to 7, wherein R⁶ is a C1-C3 alkyl.

9. The pharmaceutical composition of any one of claims 1 to 8, wherein R^{A} is -CH₂CONH₂ or -COCONH₂.

10. The pharmaceutical composition of claim 1, wherein the group V or X sPLA₂- inhibiting compound is:

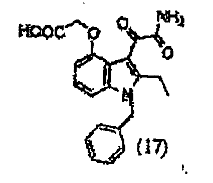

11. The pharmaceutical composition of claim 1, wherein the group V or X sPLA₂- inhibiting compound is:

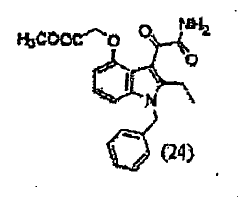

12. A group V or X sPLA₂ inhibiting compound represented by general formula (I): 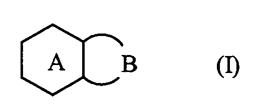 wherein
ring A is: 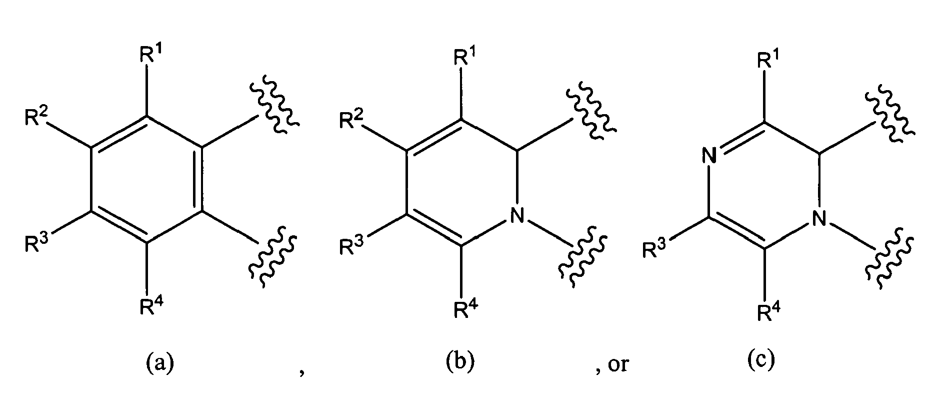 in which R¹ is a hydrogen atom or a group of formula: - (L³)-R¹¹ wherein L³ is -OCH₂-, -SCH₂-, -NH-CH₂-, -CH₂-CH₂-, -O-CH(CH₃), or -O-CH-(CH₂CH₂C₆H₅)-; and R¹¹ is -COOH, - CONHSO₂C₆H₅, -SO₃H, or -P(O)(OH₂);
R² is a hydrogen atom or a group of formula: -(L⁴)-R¹² wherein L⁴ is a group of formula: 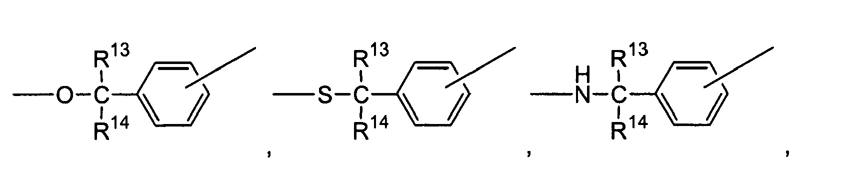 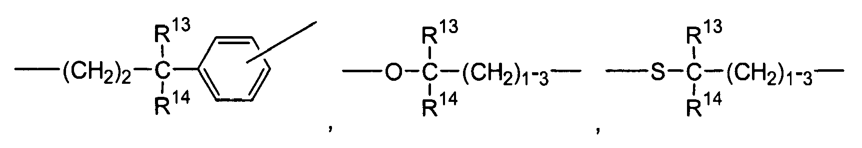 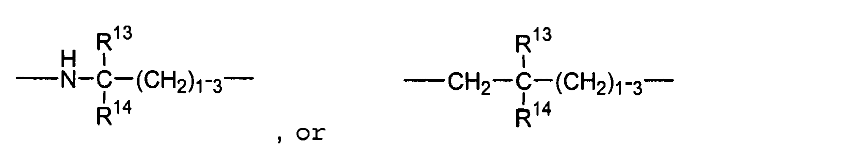 in which R¹³ and R¹⁴ is independently a hydrogen atom, a C1-C10 alkyl, a C1-C10 aralkyl, a carboxy, an alkyloxycarbonyl, or a halogen; and R¹² is -COOH, -SO₃H, or -P(O)(OH)₂; provided that R¹ and R² are not a hydrogen atom, simultaneously.
R³ is a hydrogen atom, a C1-C6 alkyl, a C3-C6 cycloalkyl, an aryl, or a heterocyclic group;
R⁴ is a hydrogen atom or a halogen;
-B- is a group of (e)-(h); 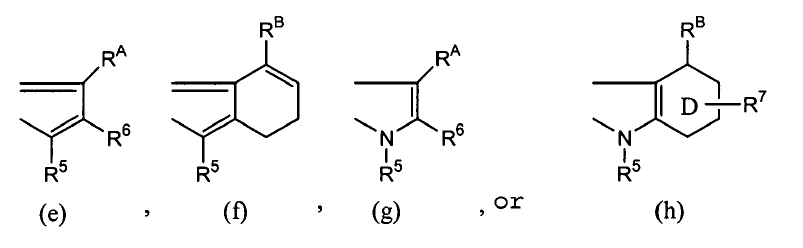 in which R⁵ is a group of -(CH₂)₁₋₆₋R¹⁵ wherein R¹⁵ is a group of formula: 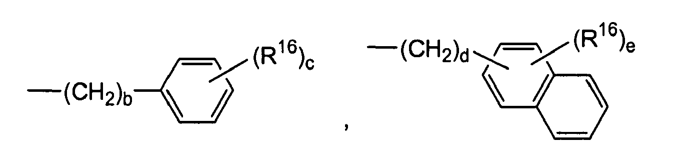 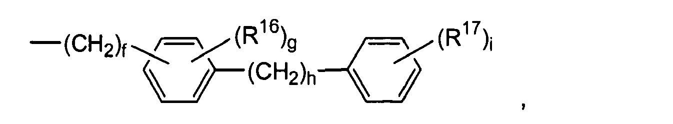 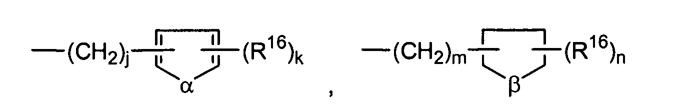 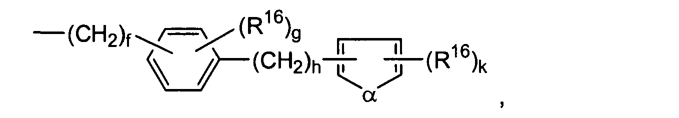 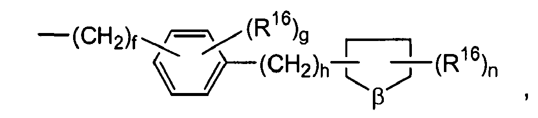 or 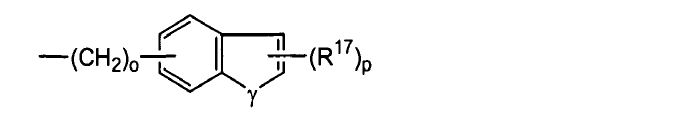 in which each b, d, f, h, j, m, and o is independently an integer of 0 to 2; each R¹⁶ and R¹⁷ is a group selected independently from a halogen, a C1-C10 alkyl, a C1-C10 alkyloxy, a C1-C10 alkylthio, an aryloxy, a phenyl, and a C1-C10 haloalkyl; α is an oxygen atom or a sulfur atom; β is -CH₂- OR (CH₂)₂-; γ is an oxygen atom or a sulfur atom; c, I, and p are an integer of 0 to 5; e is an integer of 0 to 7; g is an integer of 0 to 4; and each k and n is independently an integer of 0 to 3;
R⁶ is a C1-C3 alkyl;
R⁷ is a hydrogen atom or a non-interfering substituent;
R^{A} is -CH₂CONH₂ or -COCONH₂;
R^{B} is -CONCH₂ OR -CONHNH₂; and
ring D is a cyclohexene ring or a benzene ring; provided that, when -B- is a group of (e) or (f), then ring A is a ring of (b) or (c);
or a pharmaceutically acceptable C1-C6 alkyl ester, salt, or solvate thereof
for use in suppressing degeneration of serum lipoproteins and/or treating or preventing arteriosclerosis and/or treating or preventing an ischemic disease based on arteriosclerosis.

13. The compound of claim 12, wherein
R⁵ is a group of -CH₂-R¹⁸ wherein R¹⁸ is a group of formula: 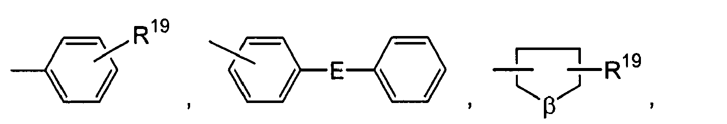 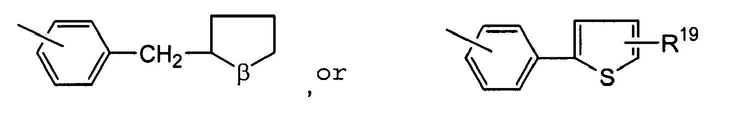 in which β is -CH₂- OR -(CH₂)₂-; R¹⁹ is a hydrogen atom, a C1-C3 alkyl, or a halogen; and E is a single bond, -CH₂-, or -O-.

14. The compound of any one of claims 12 to 13, wherein R¹ is -OCH₂COOH.

15. The compound of any one of claims 12-14, wherein R² is a hydrogen atom.


SEQUENCE LISTING

<110> Shionogi & Co., Ltd.

<120> Pharmaceutical composition for treating arteriosclerosis

<130> P64156EP00

<140>
<141>

<150> JP 2001-078569
<151> 2001-03-19

<150> JP 2001-401289
<151> 2001-12-28

<160> 3

<170> PatentIn Ver. 2.1

<210> 1
<211> 41
<212> DNA
<213> Artificial Sequence

<220>
<223> Description of Artificial Sequence: primer

<400> 1
caaagaacgc gtccaccatg aaaggcctcc tcccactggc t 41

<210> 2
<211> 38
<212> DNA
<213> Artificial Sequence

<220>
<223> Description of Artificial Sequence: primer

<400> 2
ctcgctgcgg ccgcctagga gcagaggatg ttgggaaa 38

<210> 3
<211> 57
<212> DNA
<213> Artificial Sequence

[?]

<220>
<223> Description of Artificial Sequence: primer

<400> 3
ctcgctgcgg ccgcctaatg gtgatggtga tgatgggagc agaggatgtt gggaaag 57